(19) Europäisches Patentamt
European Patent Office
Office européen des brevets

(11) Veröffentlichungsnummer: **0 033 973**
**B1**

(12) # EUROPÄISCHE PATENTSCHRIFT

(45) Veröffentlichungstag der Patentschrift: **28.09.83**

(21) Anmeldenummer: **81100906.7**

(22) Anmeldetag: **09.02.81**

(51) Int. Cl.³: **C 07 D 243/24,** C 07 D 243/26, C 07 D 243/28, C 07 D 295/14, C 07 C 103/50, A 61 K 31/55

(54) **Benzodiazepin-Derivate, Zwischenprodukte und Verfahren für ihre Herstellung, ihre Verwendung, sowie diese enthaltende Arzneimittel.**

(30) Priorität: **08.02.80 CH 1039/80**

(43) Veröffentlichungstag der Anmeldung: **19.08.81 Patentblatt 81/33**

(45) Bekanntmachung des Hinweises auf die Patenterteilung: **28.09.83 Patentblatt 83/39**

(84) Benannte Vertragsstaaten: **BE CH DE FR GB IT LI NL SE**

(56) Entgegenhaltungen:
**DE-A-2 238 579**
**DE-A-2 456 311**

(73) Patentinhaber: **F. HOFFMANN-LA ROCHE & CO. Aktiengesellschaft, CH-4002 Basel (CH)**

(72) Erfinder: **Branca, Quirico, Dr., Bettingerstrasse 7, CH-4127 Birsfelden (CH)**
Erfinder: **Fischli, Albert Eduard, Prof. Dr., Am Ausserberg 20, CH-4125 Riehen (CH)**
Erfinder: **Szente, André, Dr., Baselstrasse 70, CH-4125 Riehen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 033 973 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Benzodiazepin-Derivate, Zwischenprodukte und Verfahren für ihre Herstellung, ihre Verwendung sowie diese enthaltende Arzneimittel

Die vorliegende Erfindung betrifft Benzodiazepin-Derivate. Im Speziellen betrifft sie Benzodiazepin-Derivate der allgemeinen Formel

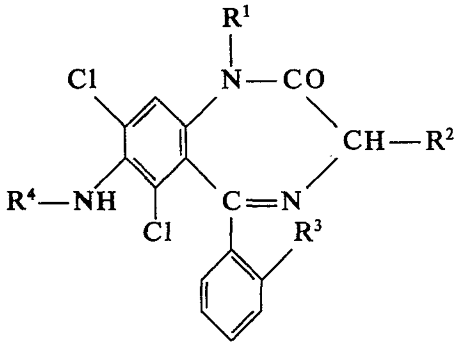

(I)

worin $R^1$ niederes Alkyl, $R^2$ Wasserstoff oder niederes Alkyl, $R^3$ Wasserstoff oder Halogen, $R^4$ Wasserstoff oder einen Rest der Formel

$R^5—CO—$

$R^6R^7N—CO—$

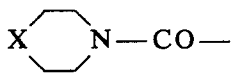

oder

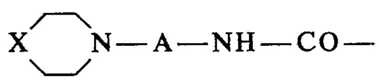

A niederes Alkylen, X ein Sauerstoff- oder Schwefelatom oder einen Rest der Formel $>N-R^8$, $R^5$ niederes Alkyl oder niederes Haloalkyl, $R^6$ Wasserstoff oder niederes Alkyl, $R^7$ niederes Alkyl und $R^8$ Wasserstoff, niederes Alkyl oder niederes Hydroxyalkyl bedeuten,
und pharmazeutisch akzeptable Säureadditionssalze davon.

Diese Verbindungen sind neu und zeichnen sich durch wertvolle pharmakodynamische Eigenschaften aus.

Gegenstand der vorliegenden Erfindung sind Verbindungen der allgemeinen Formel I und pharmazeutisch akzeptable Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine Verbindung der allgemeinen Formel I oder ein pharmazeutisch akzeptables Säureadditionssalz davon, und die Herstellung solcher Arzneimittel.

Der Ausdruck »niederes Alkyl«, für sich allein genommen oder in Kombinationen, wie »niederes Hydroxyalkyl«, »niederes Haloalkyl« und dergleichen, bezeichnet geradkettige oder verzweigte, gesättigte Kohlenwasserstoffreste mit höchstens 7, vorzugsweise höchstens 4 Kohlenstoffatomen, wie Methyl, Äthyl, n-Propyl, Isopropyl, n-Butyl, tert.-Butyl usw. Der Ausdruck »niederes Hydroxyalkyl« umfaßt Reste wie 2-Hydroxyäthyl, 3-Hydroxy-2-propyl und dergleichen. Der Ausdruck »niederes Haloalkyl« umfaßt einfach oder mehrfach halogenierte Kohlenwasserstoffreste, wie Trifluormethyl, 2-Chloräthyl, 3,3-Dichlor-2-propyl und dergleichen. Der Ausdruck »Halo« bzw. »Halogen« bedeutet Fluor, Chlor, Brom oder Jod. Der Ausdruck »niederes Alkylen« bedeutet zweiwertige, gesättigte Kohlenwasserstoffreste mit höchstens 7, vorzugsweise höchstens 4 Kohlenstoffatomen, welche geradkettig oder verzweigt sein können, wie Äthylen, 1,2-Propylen und dergleichen.

Unter den Verbindungen der Formel I sind diejenigen bevorzugt, worin $R^1$ Methyl bedeutet. Die bevorzugten Bedeutungsmöglichkeiten von $R^2$ in Formel I sind Wasserstoff und Methyl. $R^3$ in Formel I bedeutet vorzugsweise Wasserstoff, Fluor oder Chlor. Die bevorzugten Bedeutungsmöglichkeiten von $R^4$ in Formel I sind Wasserstoff und Reste der Formel

$(CH_3)_2N—CO—$

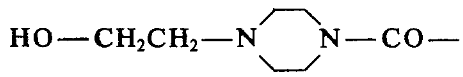

und

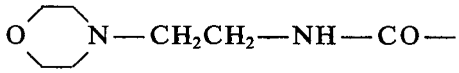

Ganz besonders bevorzugte Verbindungen im Rahmen der vorliegenden Erfindung sind:

3-[6,8-Dichlor-5-(o-fluorphenyl)-2,3-dihydro-1-methyl-2-oxo-1H-1,4-benzodiazepin-7-yl]-1,1-dimethylharnstoff,
N-[6,8-Dichlor-5-(o-fluorphenyl)-2,3-dihydro-1-methyl-2-oxo-1H-1,4-benzodiazepin-7-yl]-4-(2-hydroxyäthyl)-1-piperazin-carboxamid,
1-[6,8-Dichlor-5-(o-fluorphenyl)-2,3-dihydro-1-methyl-2-oxo-1H-1,4-benzodiazepin-7-yl]-3-(2-morpholinoäthyl)harnstoff.

Weitere im Rahmen der vorliegenden Erfindung bevorzugte, von der allgemeinen Formel I umfaßte Verbindungen sind:

7-Amino-6,8-dichlor-5-phenyl-1,3-dihydro-1-methyl-2H-1,4-benzodiazepin-2-on,
7-Amino-6,8-dichlor-5-(o-chlorphenyl)-1,3-dihydro-1,3-dimethyl-2H-1,4-benzodiazepin-2-on,
3-[6,8-Dichlor-5-(o-fluorphenyl)-2,3-dihydro-1-methyl-2-oxo-1H-1,4-benzodiazepin-7-yl]-1-methylharnstoff,
N-(6,8-Dichlor-5-(o-fluorphenyl)-2,3-dihydro-1-methyl-2-oxo-1H-1,4-benzodiazepin-7-yl]-2,2,2-trifluoracetamid und
7-Amino-6,8-dichlor-5-(o-fluorophenyl)-1,3-dihydro-1-methyl-2H-1,4-benzodiazepin-2-on.

Die Benzodiazepin-Derivate der allgemeinen Formel I und deren pharmazeutisch akzeptable Säureadditionssalze können erfindungsgemäß dadurch hergestellt werden, daß man

a) ein Benzodiazepin-Derivat der allgemeinen Formel

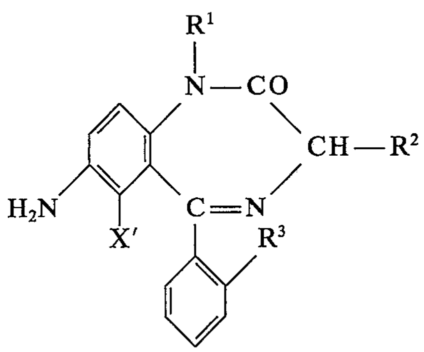

(II)

worin $R^1$, $R^2$ und $R^3$ obige Bedeutung besitzen, und X' Wasserstoff oder Chlor bedeutet, chloriert, oder

b) ein Benzodiazepin-Derivat der allgemeinen Formel

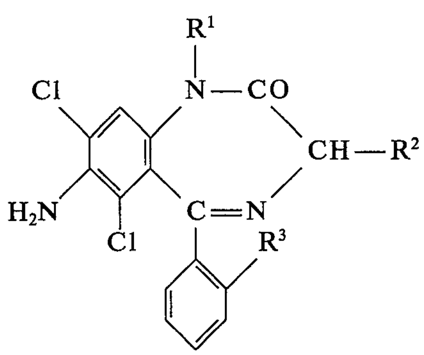

(Ia)

worin $R^1$, $R^2$ und $R^3$ obige Bedeutung besitzen, mit einer Säure der Formel $R^5$—COOH, worin $R^5$ obige Bedeutung besitzt, oder einem reaktiven Derivat davon acyliert, oder

c) ein Benzodiazepin-Derivat der obigen allgemeinen Formel Ia mit einem Halogenid der allgemeinen Formel

$R^{61}R^{71}N—CO—Y$ (III)

oder

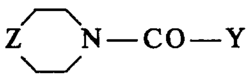 (IV)

worin Y Halogen, $R^{61}$ und $R^{71}$ je niederes Alkyl, Z ein Sauerstoff- oder Schwefelatom oder einen Rest der Formel >N—$R^{81}$ niederes Alkyl bedeuten, umsetzt, oder

d) ein Benzodiazepin-Derivat der obigen allgemeinen Formel Ia mit einem Isocyanat der allgemeinen Formel

$R^{71}—NCO$ (V)

oder

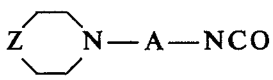 (VI)

worin A, Z und $R^{71}$ obige Bedeutung besitzen, umsetzt oder

e) ein Benzodiazepin-Derivat der allgemeinen Formel

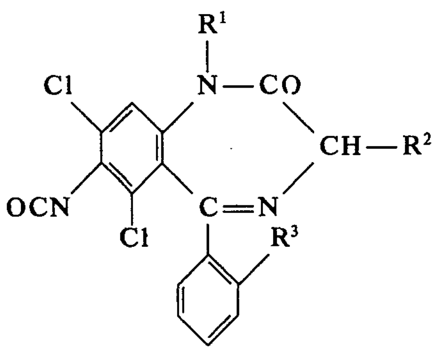 (VII)

worin $R^1$, $R^2$ und $R^3$ obige Bedeutung besitzen, mit einer Aminoverbindung der allgemeinen Formel

$R^6R^7NH$ (VIII)

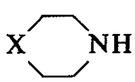 (IX)

oder

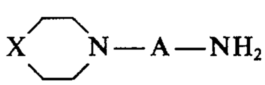 (X)

worin A, X, $R^6$ und $R^7$ obige Bedeutung besitzen, umsetzt, oder

f) aus einem Benzodiazepin-Derivat der allgemeinen Formel

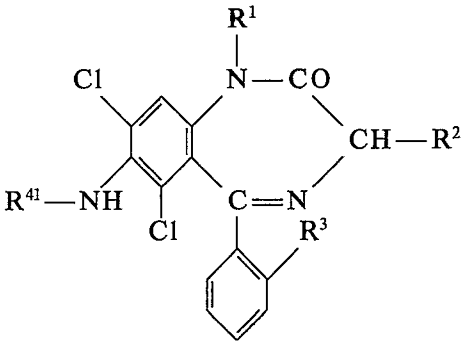

(XI)

worin $R^{41}$ eine Schutzgruppe oder einen Rest der Formel

$R^{62}R^{72}N—CO—$

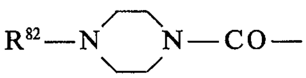

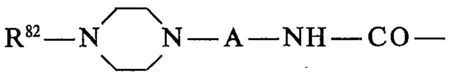

oder

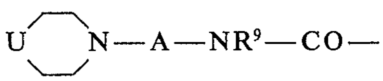

U ein Sauerstoff- oder Schwefelatom oder einen Rest der Formel $>N—R^{83}$, $R^{62}$ eine Schutzgruppe, $R^{72}$ niederes Alkyl, $R^{82}$ eine Schutzgruppe oder einen Rest der Formel —A—O—V, $R^{83}$ Wasserstoff, niederes Alkyl, niederes Hydroxyalkyl, eine Schutzgruppe oder einen Rest der Formel —A—O—V und $R^9$ und V eine Schutzgruppe bedeuten und $R^1$, $R^2$, $R^3$ und A obige Bedeutung besitzen die Schutzgruppe(n) entfernt, oder

g) ein Benzodiazepin-Derivat der allgemeinen Formel

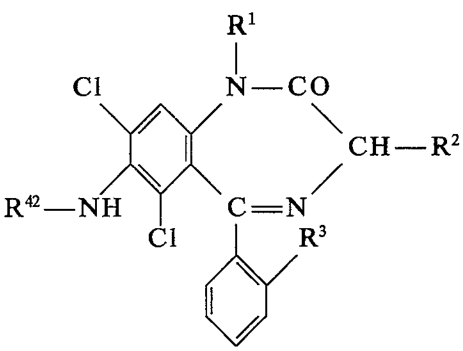

(XII)

worin $R^{42}$ einen Rest der Formel

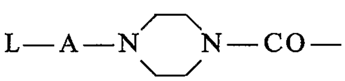

oder

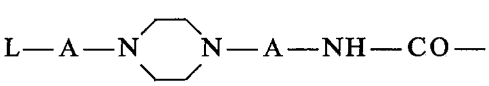

und L eine Abgangsgruppe bedeuten, und $R^1$, $R^2$, $R^3$ und A obige Bedeutung besitzen, in die entsprechende Hydroxyverbindung überführt, oder

h) ein Benzodiazepin-Derivat der allgemeinen Formel

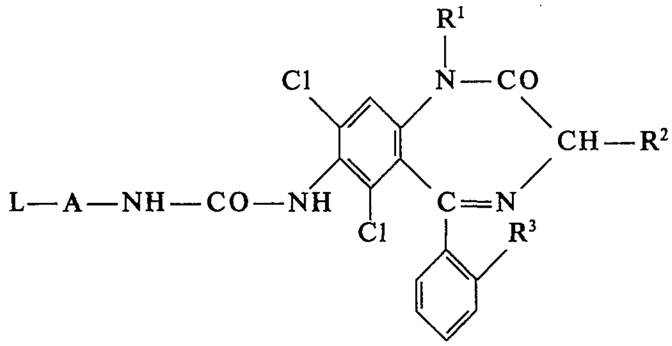

(XIII)

worin $R^1$, $R^2$, $R^3$, A und L obige Bedeutung besitzen, mit einer Verbindung der Formel

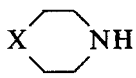

worin X obige Bedeutung besitzt, umsetzt, oder

i) ein Benzodiazepin-Derivat der allgemeinen Formel

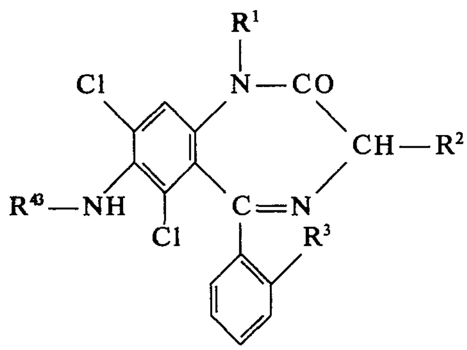

(Ib)

worin $R^{43}$ einen Rest der Formel

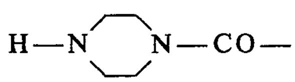

oder

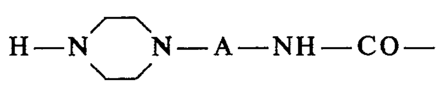

bedeutet, und $R^1$, $R^2$, $R^3$ und A obige Bedeutung besitzen, alkyliert, oder

k) ein Benzophenon-Derivat der allgemeinen Formel

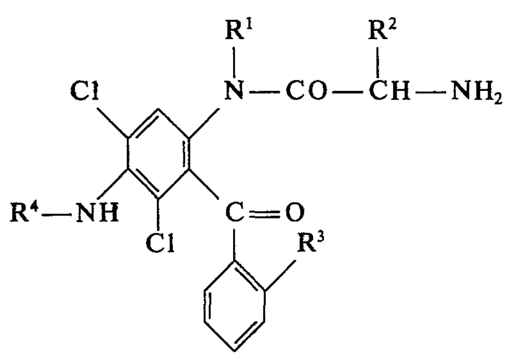

(XIV)

worin $R^1$, $R^2$, $R^3$ und $R^4$ obige Bedeutung besitzen, cyclisiert, oder

l) ein Benzodiazepin-Derivat der allgemeinen Formel I in ein pharmazeutisch akzeptables Säureadditionssalz überführt.

Nach einem ersten Verfahrensaspekt können Benzodiazepin-Derivate der allgemeinen Formel I erfindungsgemäß dadurch hergestellt werden, daß man eine Verbindung der Formel II chloriert. Als Chlorierungsmittel verwendet man zweckmäßigerweise Verbindungen wie N-Chlorsuccinimid, N-Chloracetamid und dergleichen. Als Lösungsmittel kommen in erster Linie halogenierte Kohlenwasserstoffe, wie Methylenchlorid, Dichloräthan, Chloroform, in Frage; aber auch andere, unter den Reaktionsbedingungen inerte organische Lösungsmittel, wie Acetonitril, Äther, können verwendet werden. Die Umsetzung erfolgt zweckmäßigerweise bei Temperaturen von 0° bis Raumtemperatur.

Als Chlorierungsmittel kann man auch elementares Chlor verwenden, wobei die Umsetzung zweckmäßigerweise in saurer, wäßriger Lösung, mit Chlorwasserstoff als bevorzugte Säure, erfolgt. Die Reaktion erfolgt zweckmäßigerweise bei $-10°$ bis $+10°$, vorzugsweise bei 0°.

Nach einem zweiten Verfahrensaspekt können Benzodiazepin-Derivate der allgemeinen Formel I dadurch hergestellt werden, daß man Benzodiazepin-Derivate der allgemeinen Formel Ia mit einem den Rest $R^5-CO-$ liefernden Mittel acyliert. Diese Reaktion kann mit jedem geeigneten Acylierungsmittel durchgeführt werden, beispielsweise mit einem Säureanhydrid, wie Trifluoressigsäureanhydrid, mit einem Säurehalogenid, wie Acetylchlorid. Die Reaktionsbedingungen können von jedem Fachmann je nach dem zum Einsatz vorgesehenen Acylierungsmittel leicht ausgewählt werden. Beispielsweise kann man bei Raumtemperatur oder oberhalb oder unterhalb der Raumtemperatur arbeiten.

Die Acylierung erfolgt zweckmäßigerweise in einem unter den Reaktionsbedingungen inerten organischen Lösungsmittel, beispielsweise in Acetonitril oder dergleichen, in Methylenchlorid, Dichloräthan, in Tetrahydrofuran, Dimethoxyäthan, und in Gegenwart eines säurebindenden Mittels, beispielsweise einer anorganischen Base, wie Kaliumcarbonat, Natriumcarbonat oder einer tertiären organischen Aminoverbindung, wie Triäthylamin, N-Äthyl-diisopropylamin, Chinuclidin.

Nach einem weiteren Verfahrensaspekt können Benzodiazepin-Derivate der allgemeinen Formel I erfindungsgemäß dadurch hergestellt werden, daß man ein Benzodiazepin-Derivat der allgemeinen Formel Ia mit einem Halogenid der allgemeinen Formel III oder IV umsetzt. Die Umsetzung der Verbindungen der Formeln Ia und III bzw. IV erfolgt in Gegenwart eines säurebindenden Mittels, beispielsweise einer anorganischen Base, wie Kaliumcarbonat, Natriumcarbonat usw., oder einer organischen Base, z. B. einer tertiären Aminoverbindung, wie Triäthylamin, N-Äthyl-diisopropylamin, Chinuclidin. Die Umsetzung der Verbindungen der Formeln Ia und III bzw. IV erfolgt zweckmäßigerweise bei Raumtemperatur oder unterhalb davon, sie verläuft ziemlich langsam und dauert in der Regel mehrere Tage.

Nach einem weiteren Verfahrensaspekt können Benzodiazepin-Derivate der allgemeinen Formel I erfindungsgemäß dadurch hergestellt werden, daß man ein Benzodiazepin-Derivat der allgemeinen Formel Ia mit einem Isocyanat der allgemeinen Formel V oder VI umsetzt. Diese Umsetzung erfolgt zweckmäßigerweise in einem unter den Reaktionsbedingungen inerten organischen Lösungsmittel, beispielsweise in einem halogenierten Kohlenwasserstoff, wie Methylenchlorid, Dichloräthan, Chloroform, o-Dichlorbenzol; in einem Äther, wie Tetrahydrofuran, Dioxan, Dimethoxyäthan, Diäthylenglykoldimethyläther. In manchen Fällen erweist es sich als günstig, die Reaktion in Gegenwart einer katalytisch wirkenden geringen Menge einer Base durchzuführen, beispielsweise in Gegenwart einer tertiären Aminoverbindung, wie Triäthylamin, N-Äthyl-diisopropylamin, Chinuclidin. Die Temperatur ist für die Umsetzung der Verbindungen der Formeln Ia und V bzw. VI nicht kritisch, und man kann demnach bei Raumtemperatur, unterhalb der Raumtemperatur oder oberhalb der Raumtemperatur arbeiten, beispielsweise bei Rückflußtemperatur.

Nach einem weiteren Verfahrensaspekt können die Benzodiazepin-Derivate der allgemeinen Formel I aus Benzodiazepin-Derivaten der allgemeinen Formel VII und Aminoverbindungen der allgemeinen Formel VIII, IX oder X hergestellt werden. Zweckmäßigerweise geht man dabei so vor, daß man das zum Einsatz vorgesehene Benzodiazepin-Derivat der allgemeinen Formel VII kurz oder unmittelbar vor der Umsetzung mit der Aminoverbindung der allgemeinen Formel VIII, IX oder X auf die weiter unten beschriebene Weise aus einem entsprechenden Benzodiazepin-Derivat der allgemeinen Formel Ia herstellt und das betreffende Benzodiazepin-Derivat der allgemeinen Formel VII nicht in isolierter Form in die Reaktion einbringt, sondern in der Lösung, in welcher es vorgängig aus dem entsprechenden Benzodiazepin-Derivat der allgemeinen Formel Ia hergestellt worden ist.

Zu der erwähnten, das Benzodiazepin-Derivat der allgemeinen Formel VII enthaltenden Lösung kann man dann eine Aminoverbindung der allgemeinen Formel VIII, IX oder X geben. Hierbei kann die Aminoverbindung der allgemeinen Formel VIII, IX oder X in Form einer Lösung oder auch ohne Lösungsmittel verwendet werden; falls es sich um eine bei Raumtemperatur gasförmige Aminoverbindung handelt (was beispielsweise für Methylamin zutrifft), kann man sie als Gas in die erwähnte, das Benzodiazepin-Derivat der allgemeinen Formel VII enthaltende Lösung einleiten.

Andererseits ist es auch möglich, die Aminoverbindung der allgemeinen Formel VIII, IX oder X vorzulegen, zweckmäßigerweise in Form einer Lösung, und dann die erwähnte, das Benzodiazepin-Derivat der allgemeinen Formel VII enthaltende Lösung zuzugeben.

In vielen Fällen ist es zweckmäßig, die Aminoverbindung der allgemeinen Formel VIII, IX oder X im Überschuß einzusetzen, und dies ist dann notwendig, wenn sie mehr als 1 Stickstoffatom enthält, welches zur Reaktion mit einer Isocyanatgruppe befähigt ist; dies ist beispielsweise beim Piperazin der Fall.

Als Lösungsmittel für den vorliegenden Verfahrensaspekt eignen sich verschiedene, unter den Reaktionsbedingungen inerte organische Lösungsmittel, beispielsweise halogenierte Kohlenwasserstoffe, wie Dichloräthan, Methylenchlorid, Chloroform, o-Dichlorbenzol; Äther, wie Tetrahydrofuran, Dioxan, Dimethoxyäthan, Diäthylenglykoldimethyläther.

Die Umsetzung der Verbindungen der Formeln VII und VIII bzw. IX bzw. X erfolgt zweckmäßigerweise bei Raumtemperatur oder Temperaturen unterhalb davon. Es ist noch zu beachten, daß man dann, wenn man eine Lösung des Benzodiazepin-Derivats der allgemeinen Formel VII vorlegt, die Aminoverbindung der allgemeinen Formel VIII, IX oder X innerhalb kurzer Zeit zugeben sollte, wogegen im umgekehrten Fall, d. h., wenn man die Aminoverbindung der allgemeinen Formel VIII, IX oder X vorlegt und dann die Lösung des Benzodiazepin-Derivats der allgemeinen Formel VII zugibt, die Geschwindigkeit der Zugabe keine wesentliche Rolle spielt.

Nach einem weiteren Verfahrensaspekt können Benzodiazepin-Derivate der allgemeinen Formel I erfindungsgemäß dadurch hergestellt werden, daß man aus einem Benzodiazepin-Derivat der allgemeinen Formel XI die Schutzgruppe bzw. die Schutzgruppen entfernt. Als Stickstoff-Schutzgruppen eignen sich für die Zwecke der vorliegenden Erfindung in erster Linie Acylgruppen, vorzugsweise leicht abspaltbare Alkoxycarbonyl- oder Aralkoxycarbonylgruppen, insbesondere die tert.-Butoxycarbonylgruppe, die Benzyloxycarbonylgruppe, ferner auch leicht abspaltbare Aralkylgruppen, wie die Benzylgruppe. Als Sauerstoff-Schutzgruppen eignen sich einerseits Acylgruppen oder Aralkylgruppen, wie sie soeben als Stickstoff-Schutzgruppen erwähnt worden sind, andererseits aber auch Ketal-Schutzgruppen, wie Tetrahydropyranyl, 2-Methoxy-2-propyl, Methoxymethyl, $\beta$-Methoxy-äthoxy-methyl, leicht abspaltbare Alkylgruppen, wie tert.-Butyl, oder Alkanoylgruppen, wie Acetyl.

Die Entfernung der Schutzgruppe bzw. der Schutzgruppen aus den Benzodiazepin-Derivaten der allgemeinen Formel XI erfolgt nach an sich bekannten Methoden, wobei natürlich die Natur der zu entfernenden Schutzgruppe bzw. Schutzgruppen für die Wahl der zur Anwendung gelangenden Methode bzw. Methoden in Betracht gezogen werden muß. Ebenfalls zu beachten ist natürlich, daß nur solche Methoden verwendet werden können, welche die Schutzgruppe bzw. Schutzgruppen selektiv entfernen, ohne daß andere im Molekül vorhandene Strukturelemente in Mitleidenschaft gezogen werden.

Die weiter oben als Beispiele für in Betracht kommende Schutzgruppen erwähnten Reste können je nach ihrer Natur hydrogenolytisch und/oder hydrolytisch abgespalten werden. So können z. B. die Benzyloxycarbonylgruppe und die tert.-Butoxycarbonylgruppe unter selektiven sauren Bedingungen abgespalten werden, z. B. durch Behandeln mit einem Gemisch von Bromwasserstoff und Eisessig, ebenso durch Behandlung mit Bortrifluorid oder Bortribromid in einem inerten organischen Lösungsmittel, wie Dichlormethan. Die tert.-Butoxycarbonylgruppe kann auch durch Behandeln mit Chlorwasserstoff in einem inerten organischen Lösungsmittel, wie Dioxan, Tetrahydrofuran, oder durch Behandeln mit Trifluoressigsäure abgespalten werden. Die Tetrahydropyranylgruppe läßt sich unter milden sauren Bedingungen abspalten, beispielsweise durch Behandlung mit verdünnter wäßriger Mineralsäure unter milden Bedingungen. Die tert.-Btuylgruppe kann z. B. mittels Trifluoressigsäure abgespalten werden. Die Benzylgruppe kann durch katalytische Hydrierung, z. B. über Palladium/Kohle, entfernt werden. Die Acetylgruppe kann unter milden alkalischen Bedingungen abgespalten werden, z. B. mit einer Lösung eines Natriumalkoholats im entsprechenden Alkohol (wie methanolisches Natriummethylat).

Nach einem weiteren Verfahrensaspekt können Benzodiazepin-Derivate der allgemeinen Formel I erfindungsgemäß dadurch hergestellt werden, daß man Benzodiazepin-Derivate der allgemeinen Formel XII in entsprechende Hydroxy-Verbindungen überführt. Die in Formel XII durch das Symbol L wiedergegebene Abgangsgruppe kann ein Halogenatom sein, insbesondere Chlor, Brom oder Jod; es können jedoch ohne weiteres auch äquivalente andere Abgangsgruppen verwendet werden, z. B. Arylsulfonyloxyreste, wie Tosyloxy, Alkylsulfonyloxyreste, wie Mesyloxy, quartäre Ammoniumgruppen, wie der Trimethylammoniumrest.

Die Überführung eines Benzodiazepin-Derivats der allgemeinen Formel XII in die entsprechende Hydroxyverbindung kann z. B. durch Solvolyse in einem wasserhaltigen System erfolgen, zweckmäßigerweise in einem Gemisch eines aromatischen Kohlenwasserstoffes, wie Benzol, und Wasser, in Gegenwart eines quartären Ammoniumsalzes, wie Tetrabutylammoniumbromid, und bei einer Temperatur zwischen Raumtemperatur und Rückflußtemperatur des Reaktionsgemisches.

Nach einem weiteren Verfahrensaspekt können Benzodiazepin-Derivate der allgemeinen Formel I dadurch hergestellt werden, daß man eine Verbindung der Formel XIII mit einem Amin der Formel IX umsetzt. Die in Formel XIII durch das Symbol L wiedergegebene Abgangsgruppe kann ein Halogenatom sein, insbesondere Chlor, Brom oder Jod; es können jedoch ohne weiteres auch äquivalente andere Abgangsgruppen verwendet werden, z. B. Arylsulfonyloxyreste, wie Tosyloxy, Alkylsulfonyloxyreste, wie Mesyloxy.

Die Umsetzung erfolgt zweckmäßigerweise in einem unter den Reaktionsbedingungen inerten organischen Lösungsmittel, beispielsweise in einem Äther, wie Diäthyläther, Tetrahydrofuran, Dioxan usw. oder in einem Alkohol, wie Äthanol, Äthylenglykol, in Gegenwart eines säurebindenden Mittels, beispielsweise einer anorganischen Base, wie Kaliumcarbonat, Natriumcarbonat, oder einer organischen Base, wie Triäthylamin, überschüssiges Amin der Formel IX. Ein Überschuß an Amin der Formel IX ist dann notwendig, wenn es zwei reakionsfähige Aminogruppen enthält, wie beispielsweise Piperazin. Die Reaktionstemperatur kann in einem weiten Bereich variiert werden, von 0° bis Siedetemperatur des Reaktionsgemisches, je nach Reaktivität der Abgangsgruppe L.

Nach einem weiteren Verfahrensaspekt können die Benzodiazepin-Derivate der allgemeinen Formel I dadurch hergestellt werden, daß man eine Verbindung der Formel Ib alkyliert, worunter die Einführung einer niederen Alkylgruppe oder einer niederen Hydroxyalkylgruppe zu verstehen ist. Diese Reaktion kann mit jedem geeigneten Alkylierungsmittel durchgeführt werden, beispielsweise mit einem entsprechenden Halogenid, wie Methyljodid, 2-Bromäthanol, Jodäthan, Dialkylsulfaten, wie Dimethylsulfat oder Diäthylsulfat, in Gegenwart eines säurebindenden Mittels, mit einem Aldehyd, wie Formaldehyd, Acetaldehyd, unter reduzierenden Bedingungen, mit einer entsprechenden Epoxyverbindung, wie Äthylenoxid und dergleichen.

Die Reaktionsbedingungen können von jedem Fachmann je nach dem zum Einsatz gelangenden Alkylierungsmittel leicht ausgewählt werden. Beispielsweise wird das Benzodiazepin-Derivat mit einer äquivalenten Menge Aldehyd in Ameisensäure am Rückfluß gekocht, bis kein Kohlendioxid mehr entweicht, worauf das Lösungsmittel im Vakuum eingeengt und die freie Base durch Neutralisation isoliert wird.

Nach einem weiteren Verfahrensaspekt können die Benzodiazepin-Derivate der allgemeinen Formel I dadurch hergestellt werden, daß man eine Verbindung der allgemeinen Formel XIV cyclisiert. Die Cyclisation einer Verbindung der allgemeinen Formel XIV erfolgt ziemlich leicht; sie kann gegebenenfalls durch längeres Stehenlassen herbeigeführt oder durch Anwendung von Wärme beschleunigt werden. Man kann in neutralem, alkalischem oder in saurem Milieu arbeiten; die alkalische Verfahrensführung ist jedoch bevorzugt. Die Cyclisation erfolgt zweckmäßigerweise in einem inerten organischen Lösungsmittel, z. B. in Kohlenwasserstoffen, wie Benzol, Toluol, in chlorierten Kohlenwasserstoffen, wie Chloroform, Methylenchlorid usw, in Äther, wie Dioxan. Für die Cyclisation der Verbindungen der allgemeinen Formel XIV eignen sich Temperaturen im Bereich zwischen Raumtemperatur und 150°C, natürlich unter Berücksichtigung des eingesetzten Lösungsmittels.

Die Verbindungen der allgemeinen Formel XIV müssen nicht notwendigerweise in isoliertem Zustand eingesetzt werden, und in manchen Fällen ist dies auch nicht möglich; in der Regel erweist es sich als zweckmäßig, die Verbindungen der allgemeinen Formel XIV ohne Isolierung aus dem Reaktionsgemisch, in dem sie hergestellt worden sind, direkt zu cyclisieren bzw. cyclisieren zu lassen.

Nach einem weiteren Verfahrensaspekt können die Benzodiazepin-Derivate der allgemeinen Formel I erfindungsgemäß in pharmazeutisch akzeptable Säureadditionssalze übergeführt werden. Die Herstellung von solchen pharmazeutisch akzeptablen Säureadditionssalzen erfolgt nach allgemein üblichen Methoden. Es kommen sowohl Salze mit anorganischen als auch Salze mit organischen Säuren in Betracht, beispielsweise Hydrochloride, Hydrobromide, Sulfate, Citrate, Acetate, Succinate, Methansulfonate, p-Toluolsulfonate.

Die als Ausgangsprodukte verwendeten Benzodiazepin-Derivate der allgemeinen Formel II gehören einer an sich bekannten Verbindungsklasse an, und es sind bereits diverse spezifische Vertreter dieser Verbindungsklasse in der Literatur beschrieben worden. Noch nicht spezifisch vorbeschriebene Vertreter dieser Verbindungsklasse können nach an sich bekannten und jedem Fachmann geläufigen Methoden hergestellt werden. Zweckmäßigerweise geht man von entsprechenden Nitroverbindungen der allgemeinen Formel

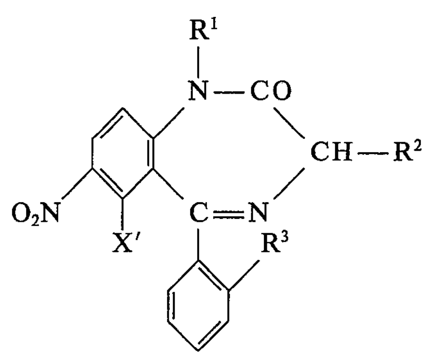

(XV)

worin $R^1$, $R^2$, $R^3$ und X' obige Bedeutung besitzen, aus; diese gehören ebenfalls einer an sich bekannten Verbindungsklasse an, von welcher verschiedene spezifische Vertreter in der Literatur beschrieben sind. Noch nicht spezifisch vorbeschriebene Vertreter dieser Verbindungsklasse können nach

Methoden hergestellt werden, welche jedem Fachmann geläufig sind und in Analogie zu denjenigen Methoden durchgeführt werden können, welche für die Herstellung der spezifisch vorbekannten Verbindungen beschrieben sind.

Die Überführung der Verbindungen der Formel XV in entsprechende Verbindungen der Formel II erfolgt durch Reduktion der Nitrogruppe, zweckmäßigerweise mittels Zinn(II)chlorid, Zink, katalytisch angeregtem Wasserstoff usw. Falls das Symbol X' in Formel XV für Wasserstoff steht und eine Verbindung der Formel II gewünscht wird, worin X' Chlor bedeutet, muß anschließend an die soeben erwähnte Reduktion noch eine Chlorierung durchgeführt werden; diese Chlorierung erfolgt vorzugsweise mittels elementarem Chlor in saurer wäßriger Lösung, wobei als Säure zweckmäßigerweise Chlorwasserstoff verwendet wird.

Die als Ausgangsprodukte verwendeten Benzodiazepin-Derivate der allgemeinen Formel VII können — wie bereits weiter oben erwähnt — aus entsprechenden Benzodiazepin-Derivaten der allgemeinen Formel Ia hergestellt werden, und zwar durch Umsetzung mit Phosgen. Dabei geht man zweckmäßigerweise so vor, daß man eine Lösung von Phosgen in einem unter den Reaktionsbedingungen inerten organischen Lösungsmittel vorlegt und dann unter Kühlen eine Lösung eines Benzodiazepin-Derivats der allgemeinen Formel Ia zugibt, hierauf einige Zeit zum Rückfluß erhitzt, dann wieder abkühlt und schließlich die erhaltene Lösung mit einer tertiären organischen Aminoverbindung, wie Triäthylamin, basisch oder mindestens neutral stellt. Die erhaltene Lösung, enthaltend ein Benzodiazepin-Derivat der allgemeinen Formel VII, kann unter Feuchtigkeitsausschluß und in der Kälte während mehrerer Stunden aufbewahrt werden; sie wird — wie weiter oben ausgeführt — ohne Isolierung des darin enthaltenen Benzodiazepin-Derivats der allgemeinen Formel VII direkt weiterverarbeitet.

Die Verbindungen der allgemeinen Formel VII sind ebenfalls Gegenstand der vorliegenden Erfindung.

Zur Herstellung von Verbindungen der Formel XI, worin $R^{41}$ eingangs erwähnte Bedeutung hat, kann man eine Verbindung der allgemeinen Formel

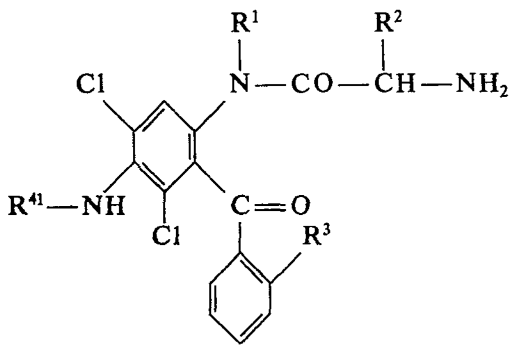

(XVI)

cyclisieren (in Analogie zur Cyclisierung von Verbindungen der Formel XIV; siehe weiter oben).

Die Verbindungen der allgemeinen Formel XI sind ebenfalls Gegenstand der vorliegenden Erfindung.

Verbindungen der allgemeinen Formel XI können aber auch nach an sich bekannten Methoden aus Verbindungen der Formeln VII oder Ia hergestellt werden, wobei natürlich die Natur der Schutzgruppe bzw. Schutzgruppen, deren Anwesenheit in der herzustellenden Verbindung der Formel XI erwünscht ist, für die Wahl der zur Anwendung gelangenden Methode bzw. Methoden in Betracht gezogen werden muß.

Zur Herstellung einer Verbindung der Formel XI, worin $R^{41}$ einen Rest der Formel

$R^{62}R^{72}N—CO—$

oder

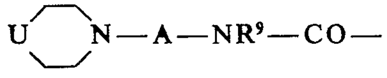

worin U obige Bedeutung besitzt, wobei aber $R^{83}$ nur niederes Alkyl, eine Schutzgruppe oder ein Rest der Formel $-A-O-V$ ist, bedeutet, kann man ein Benzodiazepin-Derivat der Formel Ia mit einem entsprechenden Carbamoylhalogenid umsetzen (in Analogie zu der weiter oben beschriebenen Methode für die Herstellung von Verbindungen der Formel I aus Verbindungen der Formeln Ia und III).

Zur Herstellung von Verbindungen der Formel XI, worin $R^{41}$ einen Rest der Formel

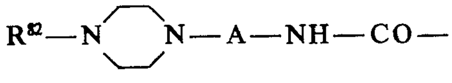

bedeutet, kann man ein Benzodiazepin-Derivat der allgemeinen Formel Ia mit einem entsprechenden Isocyanat umsetzen (in Analogie zur weiter oben beschriebenen Methode für die Herstellung von Verbindungen der Formel I aus Verbindungen der Formeln Ia und VI). Eine andere Möglichkeit besteht in der Umsetzung eines Benzodiazepin-Derivats der allgemeinen Formel VII mit einem entsprechenden Amin (in Analogie zu der weiter oben beschriebenen Methode für die Herstellung von Verbindungen der Formel I aus Verbindungen der Formeln VII und X); dabei ist jedoch zu beachten, daß in diesem Fall als Schutzgruppe (V, $R^{82}$) eine Acylgruppe nicht in Betracht kommt.

Zur Herstellung von Verbindungen der Formel XI, worin $R^{41}$ einen Rest der Formel

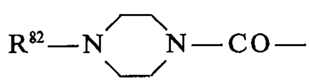

bedeutet, kann man ein Benzodiazepin der allgemeinen Formel Ia mit einem entsprechenden Carbamoylhalogenid umsetzen (in Analogie zu der weiter oben beschriebenen Methode für die Herstellung von Verbindungen der Formel I aus Verbindungen der Formel Ia und III). Eine andere Möglichkeit besteht in der Umsetzung eines Benzodiazepin-Derivats der allgemeinen Formel VII mit einem entsprechenden Amin (in Analogie zur weiter oben beschriebenen Methode für die Herstellung von Verbindungen der Formel I aus Verbindungen der Formeln VII und IX), wobei wiederum als Schutzgruppe (V, $R^{82}$) eine Acylgruppe nicht in Betracht kommt.

Verbindungen der Formel XII können nach an sich bekannten Methoden aus Aminobenzodiazepin-Derivaten der allgemeinen Formel Ia hergestellt werden, und zwar durch Umsetzung mit einem entsprechenden Carbamoylchlorid (in Analogie zur weiter oben beschriebenen Methode für die Herstellung von Verbindungen der Formel I aus Verbindungen der Formeln Ia und IV) oder durch Umsetzung mit einem entsprechenden Isocyanat (in Analogie zur weiter oben beschriebenen Methode für die Herstellung von Verbindungen der Formel I aus Verbindungen der Formeln Ia und VI).

Verbindungen der Formel XIII können nach an sich bekannten Methoden aus Aminobenzodiazepin-Derivaten der allgemeinen Formel Ia hergestellt werden, und zwar durch Umsetzung mit einem entsprechenden Isocyanat (in Analogie zur weiter oben beschriebenen Methode für die Herstellung von Verbindungen der Formel I aus Verbindungen der Formel Ia und V). Die Verbindungen der Formel XII und XIII sind ebenfalls Gegenstand der vorliegenden Erfindung.

Verbindungen der allgemeinen Formel XIV können nach an sich bekannten Methoden hergestellt werden, wobei man teilweise in Analogie zu Methoden arbeiten kann, welche weiter oben im Zusammenhang mit gewissen Verfahren zur Herstellung von Verbindungen der Formeln I und XI beschrieben sind. Als Ausgangsprodukte zur Herstellung der Verbindungen der Formel XIV dienen zweckmäßigerweise Benzophenon-Derivate der allgemeinen Formel

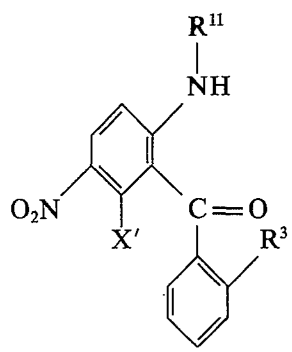

(XVII)

worin $R^3$ und X' obige Bedeutung besitzen und $R^{11}$ Wasserstoff oder niederes Alkyl bedeutet.

Beispielsweise kann man ein Benzophenon-Derivat der allgemeinen Formel XVII zunächst in eine Verbindung der allgemeinen Formel

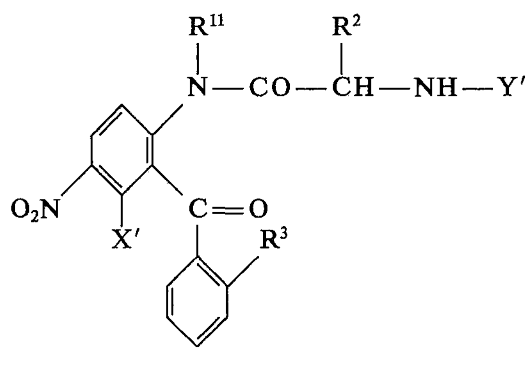

(XVIII)

worin $R^{11}$, $R^2$, $R^3$ und X' die obige Bedeutung besitzen, und Y' eine Schutzgruppe bedeutet, überführen, worauf — falls $R^{11}$ in Formeln XVII und XVIII Wasserstoff bedeutet — das Stickstoffatom alkyliert, die Nitrogruppe zur Aminogruppe reduziert und die erhaltene Aminoverbindung chloriert wird. Als Schutzgruppen (vgl. Symbol Y' in Formel XVIII) eignen sich in erster Linie Acylgruppen, vorzugsweise leicht abspaltbare Alkoxycarbonyl- oder Aralkoxycarbonylgruppen, insbesondere die Benzyloxycarbonylgruppe; zur Herstellung der Verbindungen der Formel XVIII aus den Benzophenonderivaten der Formel XVII verwendet man demnach zweckmäßigerweise entsprechende Acylaminoalkanoylhalogenide, wie Carbobenzoxyglycinchlorid, Carbobenzoxyalaninchlorid, Carbobenzoxy-$\alpha$-aminobuttersäurechlorid. Falls eine N-Alkylierung durchgeführt werden muß, so erfolgt dies nach an sich bekannten Methoden, beispielsweise mittels Methyljodid in Gegenwart einer Base, wie Kaliumcarbonat, und in einem geeigneten, unter den Reaktionsbedingungen inerten Lösungsmittel, wie Aceton. Die Reduktion der Nitro- zur Aminogruppe erfolgt zweckmäßigerweise mit Zinn(II)-chlorid. Die Chlorierung erfolgt zweckmäßigerweise in Analogie zur Herstellung von Verbindungen der Formel Ia aus Benzodiazepin-Derivaten der Formel II.

Die auf die vorstehend beschriebene Weise erhaltenen 5-Aminobenzophenon-Derivate der allgemeinen Formel

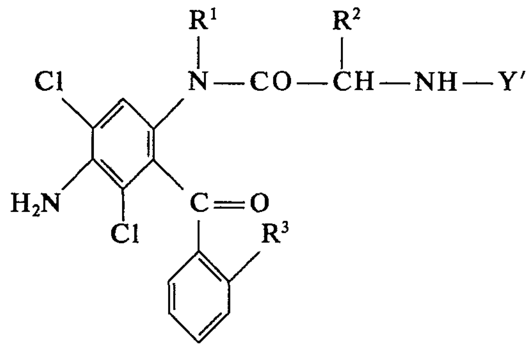

(XIX)

worin $R^1$, $R^2$, $R^3$ und Y' obige Bedeutung besitzen, werden anschließend in entsprechende Verbindungen der allgemeinen Formel

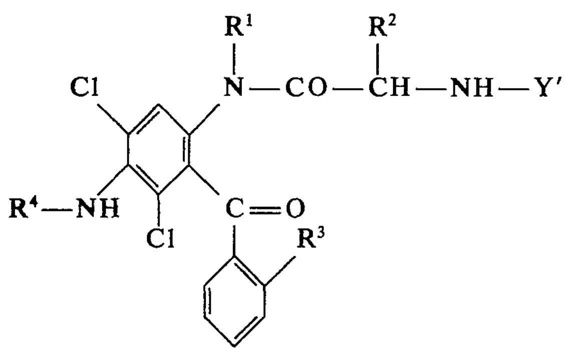

(XX)

worin $R^1$, $R^2$, $R^3$, $R^4$ und Y' obige Bedeutung besitzen, übergeführt, was beispielsweise so bewerkstelligt werden kann, daß die Verbindung der Formel XIX in Analogie zu weiter oben beschriebenen Methoden mit einem Halogenid der allgemeinen Formel III oder IV oder einem Isocyanat der allgemeinen Formel V oder VI umgesetzt wird oder daß die Verbindung der allgemeinen Formel XIX — in Analogie zur weiter oben beschriebenen Methode für die Herstellung der Verbindungen der Formel VII — in das entsprechende Isocyanat übergeführt wird, welches dann mit einer Aminoverbindung der allgemeinen Formel VIII, IX oder X umgesetzt wird, in Analogie zur weiter oben beschriebenen Methode zur Herstellung von Verbindungen der Formel I aus Verbindungen der Formel VII und VIII, IX bzw. X, oder daß die Verbindung der Formel XIX mit einer Säure der Formel $R^5-COOH$, worin $R^5$ die obige Bedeutung besitzt oder einem reaktiven Derivat davon acyliert wird, in Analogie zur Herstellung von Verbindungen der Formel I aus Verbindungen der Formel Ia und $R^5COOH$, oder einem reaktiven Derivat davon.

Durch Abspaltung der Schutzgruppe (Y') aus der Verbindung der Formel XX erhält man dann die entsprechende Verbindung der Formel XIV.

Es ist auch möglich, die Verbindung der allgemeinen Formel XIX in eine Verbindung der allgemeinen Formel

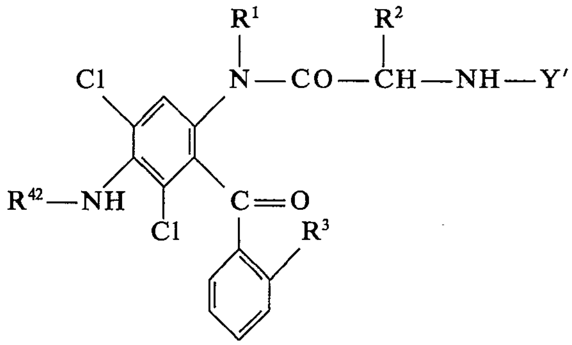

(XXI)

worin $R^1$, $R^2$, $R^3$, $R^{42}$ und Y' obige Bedeutung besitzen, überzuführen (in Analogie zur weiter oben beschriebenen Methode für die Herstellung der Verbindungen der allgemeinen Formel XII), hierauf die Verbindung der Formel XXI in die entsprechende Hydroxyverbindung überzuführen (in Analogie zur weiter oben beschriebenen Methode für die Herstellung von Verbindungen der Formel I aus Verbindungen der Formel XII) und hierauf durch Abspaltung der Schutzgruppe (Y') zu einer entsprechenden Verbindung der Formel XIV zu gelangen; weiterhin ist es möglich, die Verbindung der Formel XIX in eine Verbindung der allgemeinen Formel

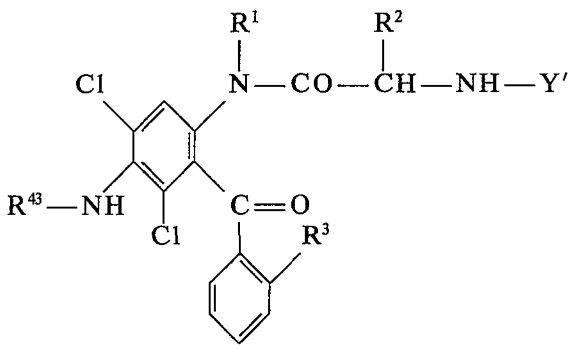

(XXa)

worin $R^1$, $R^2$, $R^3$, $R^{43}$ und Y' obige Bedeutung besitzen, überzuführen, worauf man dann durch Alkylierung (in Analogie zur weiter oben beschriebenen Methode für die Herstellung von Verbindungen der Formel I aus Verbindungen der Formel Ib) und anschließende Abspaltung der Schutzgruppe (Y') zu einer entsprechenden Verbindung der Formel XIV gelangt.

Weiterhin ist es möglich, die Verbindung der allgemeinen Formel IXI in eine Verbindung der allgemeinen Formel

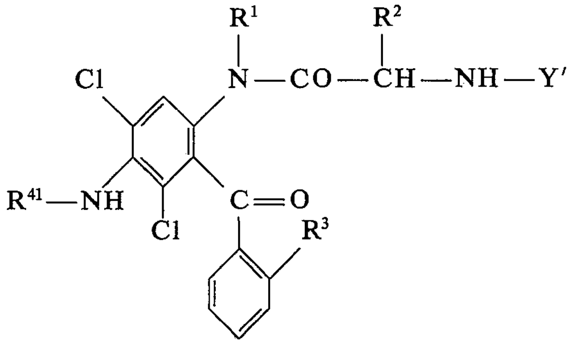

(XXII)

worin $R^1$, $R^2$, $R^3$, $R^{41}$ und Y' obige Bedeutung besitzen, überzuführen, wobei man in Analogie zu den weiter oben beschriebenen Methoden zur Herstellung der Verbindungen der Formel XI vorgehen kann; zu Verbindungen der allgemeinen Formel XIV gelangt man aus Verbindungen der allgemeinen Formel XXII dadurch, daß man die Schutzgruppe Y' und — vorgängig oder im gleichen Arbeitsgang — die weitere bzw. die weiteren im Molekül vorhandene bzw. vorhandenen Schutzgruppe bzw. Schutzgruppen abspaltet.

Eine weitere Möglichkeit zur Herstellung von Verbindungen der Formel XIV besteht darin, daß man ein Nitrobenzophenon-Derivat der allgemeinen Formel XVII in eine Verbindung der allgemeinen Formel

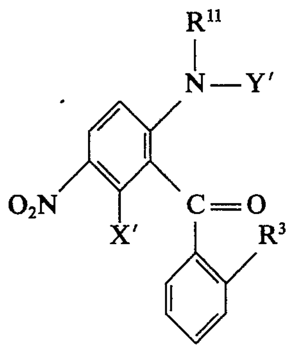

(XXIII)

worin $R^{11}$, $R^3$, X' und Y' obige Bedeutung besitzen, überführt, hierauf – falls $R^{11}$ in Formel XXIII Wasserstoff bedeutet – das Stickstoffatom alkyliert, die Nitrogruppe reduziert und das erhaltene 5-Aminobenzophenon-Derivat chloriert. Die auf die soeben beschriebene Weise erhaltenen Verbindungen entsprechen der allgemeinen Formel

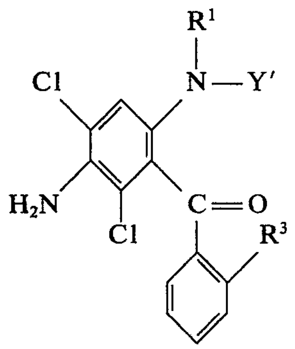

(XXIV)

worin $R^1$, $R^3$ und Y' obige Bedeutung besitzen.

Eine Verbindung der obigen Formel XXIV kann sodann in eine Verbindung der Formel

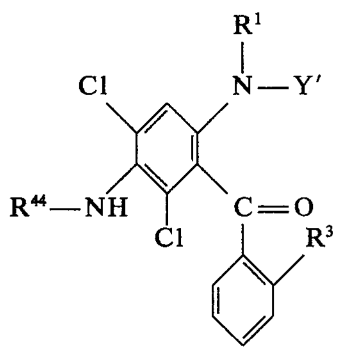

(XXV)

worin $R^{44}$ einen Rest der Formel

$R^5—CO—$

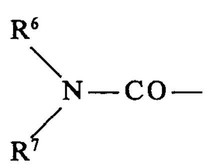

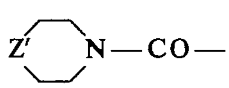

oder

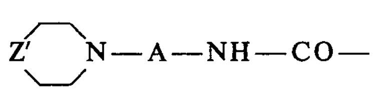

Z' ein Sauerstoff- oder Schwefelatom oder einen Rest der Formel $R^{84}-N <$ und $R^{84}$ niederes Alkyl bedeuten und $R^1$, $R^3$, $R^5$, $R^6$, $R^7$, A und Y' obige Bedeutung haben, überführt werden, beispielsweise durch Umsetzung mit einem Halogenid der allgemeinen Formel III oder IV bzw. einem Isocyanat der allgemeinen Formel V oder VI (in Analogie zur oben beschriebenen Methode für die Herstellung von Verbindungen der Formel I aus Verbindungen der Formel Ia) bzw. mit einer Säure der Formel $R^5-COOH$, worin $R^5$ obige Bedeutung besitzt, oder einem reaktiven Derivat davon (in Analogie zur oben beschriebenen Methode zur Herstellung von Verbindungen der Formel I aus Verbindungen der Formel Ia und $R^5-COOH$ oder einem reaktiven Derivat davon), oder durch Überführung in das entsprechende Isocyanat (in Analogie zum oben beschriebenen Verfahren für die Herstellung von Verbindungen der Formel VII) und anschließende Umsetzung dieses Isocyanats mit einer Aminoverbindung der allgemeinen Formel VIII, IX oder X (in Analogie zu dem weiter oben beschriebenen Verfahren für die Herstellung von Benzodiazepin-Derivaten der allgemeinen Formel I aus Verbindungen der allgemeinen Formel VII). Durch Abspaltung der Schutzgruppe (Y') aus der Verbindung der allgemeinen Formel XXV erhält man ein Benzophenon-Derivat der allgemeinen Formel

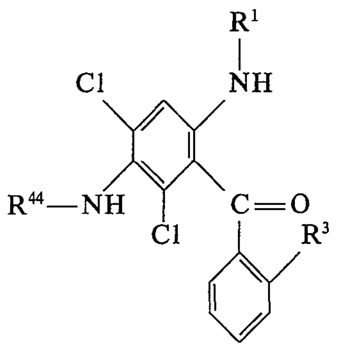

(XXVI)

worin $R^1$, $R^3$ und $R^{44}$ obige Bedeutung besitzen.

Verbindungen der allgemeinen Formel XXVI können nach einer Vielzahl von verschiedenen, an sich bekannten Methoden in entsprechende Verbindung der allgemeinen Formel XIV übergeführt werden, beispielsweise durch Umsetzung mit einem entsprechenden $\alpha$-Halogenalkanoylhalogenid und Behandlung der erhaltenen Verbindung mit Ammoniak, durch Behandlung mit einem am Stickstoffatom eine geeignete Schutzgruppe tragenden entsprechenden $\alpha$-Aminoacylierungsmittel (beispielsweise mit einem entsprechenden $\alpha$-Benzyloxycarbonylaminoalkanoylhalogenid, wie Carbobenzoxyglycinchlorid) und anschließende Abspaltung der Schutzgruppe, durch Überführung in ein entsprechendes $\alpha$-Azidoalkanoylderivat (beispielsweise in eine Azidoacetylderivat) und anschließende Reduktion usw.

Verbindungen der Formel XXIV können andererseits auch in entsprechende Verbindungen der Formel

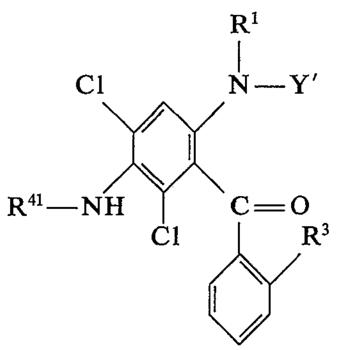

(XXVII)

worin $R^1$, $R^3$, $R^{41}$ und Y' obige Bedeutung besitzen, übergeführt werden (beispielsweise in Analogie zu den weiter oben beschriebenen Methoden für die Herstellung von Benzodiazepin-Derivaten der allgemeinen Formel XI), worauf dann die Schutzgruppe Y' entfernt wird; selbstverständlich müssen die verschiedenen Schutzgruppen in der Verbindung der Formel XXVII so beschaffen sein, daß die Schutzgruppe Y' entfernt werden kann, ohne daß eine Abspaltung der anderen Schutzgruppe bzw. Schutzgruppen im Molekül erfolgt. Die erhaltene Verbindung wird dann nach an sich bekannten Methoden in eine entsprechende Verbindung der obigen Formel XXII übergeführt, wobei man in Analogie zu der weiter oben beschriebenen Methode für die Herstellung der Verbindungen der Formel XVIII aus den Verbindungen der Formel XVII verfahren kann; die Überführung der Verbindungen der Formel XXII in entsprechende Verbindungen der Formel XIV ist bereits weiter oben erläutert worden.

Wie bereits weiter oben aufgeführt, ist es nicht notwendig (und in manchen Fällen auch nicht möglich), die Verbindungen der allgemeinen Formel XIV zu isolieren; vielmehr erweist es sich in der Regel als zweckmäßig, diese Verbindungen ohne Isolierung aus dem Reaktionsgemisch, in dem sie hergestellt worden sind, direkt zu cyclisieren bzw. cyclisieren zu lassen.

Die Verbindungen der allgemeinen Formel XIV sind ebenfalls Gegenstand der vorliegenden Erfindung.

Verbindungen der Formel XVI können durch Entfernen der Schutzgruppe Y' aus einer Verbindung der Formel XXII erhalten werden; selbstverständlich müssen die verschiedenen Schutzgruppen in der Verbindung der Formel XVI so beschaffen sein, daß die Schutzgruppe Y' entfernt werden kann, ohne daß eine Abspaltung der anderen Schutzgruppe bzw. Schutzgruppen im Molekül erfolgt.

Überraschenderweise hat es sich gezeigt, daß die Benzodiazepin-Derivate der eingangs definierten allgemeinen Formel I keine oder nur sehr geringe Wirkungen auf das zentrale Nervensystem entfalten, wogegen sie ausgeprägte aldosteronantagonistische Eigenschaften aufweisen. Diese aldosteronantagonistischen Eigenschaften lassen sich, wie nachstehend erläutert, an adrenalekomierten Ratten nachweisen.

Verabreicht man adrenalektomierten Ratten Aldosteron, so beobachtet man — im Vergleich zu unbehandelten Tieren — eine ausgeprägte Verminderung der Natrium-Ausscheidung (Natrium-Retention), eine erhöhte Kalium-Ausscheidung (Kalium-Exkretion) sowie eine Verminderung des ausgeschiedenen Harnvolumens. Gibt man den Tieren vor der Behandlung mit Aldosteron Verbindungen der Formel I, so beobachtet man — im Vergleich zu nur mit Aldosteron behandelten Tieren (Kontrolltiere) — eine ausgeprägte Erhöhung der Natrium-Ausscheidung (das heißt: die durch Aldosteron bewirkte Natrium-Retention wird antagonisiert), wogegen die Kalium-Exkretion und das Harnvolumen in geringerem Ausmaß beeinflußt werden.

Der Standardversuch wird wie folgt durchgeführt:

Weibliche Holtzmann-Ratten (150—180 g) werden 70 bis 74 Stunden vor Versuchsbeginn bilateral adrenalektomiert. Nach der Operation erhalten die Tiere ein gebräuchliches Ratten-Trockenfutter und 0,9%ige Kochsalzlösung zum Trinken. 16—17 Stunden vor Versuchsbeginn wird den Tieren das Futter entzogen, wogegen sie nach wie ad libitum 0,9%ige Kochsalzlösung trinken können. Bei Versuchsbeginn wird den Tieren die auf Aldosteronantagonismus zu prüfende Substanz mittels einer Magensonde verabreicht. 30 Minuten später erhalten die Tiere eine subcutane Injektion von 4 mmg/kg Aldosteron. Nach weiteren 90 Minuten werden die Harnblasen der Tiere durch sorgfältiges suprapubisches Drücken entleert, worauf die Tiere ohne Nahrung und ohne Getränk einzeln in Metabolismuskäfige verbracht werden. Der Urin der Tiere wird dann während 3 Stunden gesammelt, worauf ihre Harnblasen nochmals entleert werden. Der spontan ausgeschiedene Harn und der am Schluß des Versuches durch Ausdrücken der Harnblasen erhaltene Restharn werden in graduierten Zentrifugengläsern gesammelt. Natrium- und Kalium-Konzentrationen des Harns werden mit einem Flammenphotometer bestimmt.

In der nachfolgenden Tabelle werden die Resultate dargestellt, welche mit repräsentativen Vertretern der durch die allgemeine Formel I definierten Verbindungsklasse in dem vorstehend geschilderten Versuch erhalten worden sind. Angegeben werden in dieser Tabelle für jede der darin figurierenden Verbindungen die verabreichte Dosis (in mg/kg p.o.) sowie die prozentuale Veränderung des Harnvolumens, der Natrium-Ausscheidung und der Kalium-Ausscheidung im Vergleich zu den Kontrolltieren (das heißt im Vergleich zu den nur mit Aldosteron behandelten Tieren). Außerdem enthält die Tabelle Angaben über die akute Toxizität der untersuchten Verbindungen (DL 50 in mg/kg bei einmaliger oraler Verabreichung an Mäuse).

| $R^1$ | $R^2$ | $R^3$ | $R^4$ | Dosis, mg/kg p. o. | Volumen | ($Na^+$) | ($K^+$) | DL 50, mg/kg p. o. |
|---|---|---|---|---|---|---|---|---|
| | | | | | in % bez. auf Kontrolltiere | | | |
| $CH_3$ | H | F | $(CH_3)_2N—CO—$ | 1 | 152 | 330 | 160 | 2500 |
| $CH_3$ | H | F | 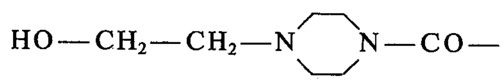 | 1 | 173 | 300 | 120 | >5000 |
| $CH_3$ | H | F | 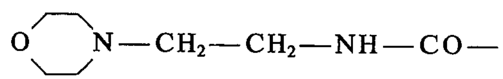 | 1 | 183 | 254 | 91 | 5000 |

Die Benzodiazepin-Derivate der allgemeinen Formel I und ihre pharmazeutisch akzeptablen Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Benzodiazepin-Derivate der allgemeinen Formel I und ihre pharmazeutisch akzeptablen Säureadditionssalze mit pharmazeutisch inerten, anorganischen oder organischen Excipientien verarbeitet werden. Als solche Excipientien kann man für Tabletten, Dragées und Hargelatinekapseln z. B. Lactose, Maisstärke oder Derivate davon, Talk, Stearinsäure oder deren Salze verwenden.

Für Weichgelatinekapseln eignen sich als Excipientien z. B. vegetabile Öle, Wachse, Fette, halbfeste und flüssige Polyole; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z. B. Wasser, Polyole, Saccharose, Invertzucker, Glukose.

Für Injektionslösungen eignen sich als Excipientien z. B. Wasser, Alkohole, Polyole, Glyzerin, vegetabile Öle.

Für Suppositorien eignen sich als Excipientien z. B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt, sind Arzneimittel, enthaltend eine oder mehrere Verbindungen der allgemeinen Formel I oder pharmazeutisch akzeptable Säureadditionssalze davon, ebenfalls Gegenstand der vorliegenden Erfindung, weiterhin auch ein Verfahren zur Herstellung solcher Arzneimittel, welches dadurch gekennzeichnet ist, daß man eine oder mehrere Verbindungen der allgemeinen Formel I oder pharmazeutisch akzeptable Säureadditionssalze davon in eine galenische Darreichungsform bringt. Die Benzodiazepin-Derivate der allgemeinen Formel I und pharmazeutisch akzeptable Säureadditionssalze davon können bei der Bekämpfung bzw. Verhütung von Krankheiten, insbesondere bei der Bekämpfung bzw. Verhütung von Herzversagen, von hepatitischer Aszites, von primärem Aldosteronismus und von essentieller Hypertonie verwendet werden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von 20 mg bis 1500 mg angemessen sein.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

### Beispiel 1

Eine gerührte Suspension von 5 g (0,019 Mol) 7-Amino-5-phenyl-1,3-dihydro-1-methyl-2H-1,4-benzodiazepin-2-on in 70 ml Methylenchlorid wird bei 0° mit 5,83 g (0,044 Mol) N-Chlorsuccinimid versetzt. Anschließend wird das Reaktionsgemisch während 27 Stunden bei Raumtemperatur gerührt und mit Methylenchlorid verdünnt. Die organische Phase wird mit 2N-Natriumcarbonatlösung gewaschen, getrocknet und eingedampft. Der Rückstand wird an 20 g Silicagel mit Methylenchlorid/Essigester (4 : 1) chromatographiert, wobei man 7-Amino-6,8-dichlor-5-phenyl-1,3-dihydro-1-methyl-2H-1,4-benzodiazepin-2-on erhält. Nach Umkristallisieren aus Methylenchlorid/Essigester weist das Produkt einen Smp. von 228° auf.

### Beispiel 2

Eine Lösung von 50 g (0,175 Mol) 7-Amino-5-(o-fluorphenyl)-1,3-dihydro-1-methyl-2H-1,4-benzodiazepin-2-on in 700 ml Methylenchlorid wird bei Raumtemperatur mit 27 g N-Chlorsuccinimid versetzt und 1 Stunde gerührt. Anschließend gibt man noch weitere 27 g N-Chlorsuccinimid hinzu und rührt 17 Stunden bei Raumtemperatur. Das Reaktionsgemisch wird mit Methylenchlorid verdünnt, die organische Phase wird mit 2N-Natriumcarbonatlösung gewaschen, getrocknet und eingedampft. Der Rückstand ergibt nach Chromatographie an 200 g Silicagel mit Methylenchlorid als Elutionsmittel 7-Amino-6,8-dichlor-5-(o-fluorphenyl)-1,3-dihydro-1-methyl-2H-1,4-benzodiazepin-2-on, das nach Umkristallisieren aus Methylenchlorid/Essigester bei 234° schmilzt.

## Beispiel 3

Zu 3,6 g (10,2 mMol) 7-amino-6,8-dichlor-5-(o-fluorphenyl)-1,3-dihydro-1-methyl-2H-1,4-benzodiazepin-2-on und 12,2 g Natriumcarbonat in 50 ml Tetrahydrofuran gibt man unter Rühren bei 0° eine Lösung von 16,7 ml Trifluoressigsäureanhydrid in 10 ml Tetrahydrofuran über einen Zeitraum von 5 Minuten hinzu. Nach 20 Minuten wird das Reaktionsgemisch in Chloroform aufgenommen, mit Wasser gewaschen, getrocknet und eingedampft. Der aus Äther umkristallisierte Rückstand ergibt N-[6,8-Dichlor-5-(o-fluorphenyl)-2,3-dihydro-1-methyl-2-oxo-1H-1,4-benzodiazepin-7-yl]-2,2,2-trifluoroacetamid vom Smp. 238—240°.

## Beispiel 4

a) 5 g (14,25 mMol) 7-Amino-6,8-dichlor-5-(o-fluorphenyl)-1,3-dihydro-1-methyl-2H-1,4-benzodiazepin-2-on werden in 325 ml 1,2-Dichloräthan unter Rückfluß in Lösung gebracht. Die noch heiße Lösung wird anschließend unter Rühren und Eiskühlen derart zu 3,37 g (37,65 mMol) Phosgen in 50 ml eisgekühltem 1,2-Dichloräthan getropft, daß die Reaktionstemperatur 20° nicht übersteigt. Man erhitzt das Reaktionsgemisch 1 Stunde unter Rückfluß, läßt 80 ml Dichloräthan abdestillieren und gibt 80 ml frisches Dichloräthan hinzu. Unter Eiskühlen wird Argon direkt in die Reaktionslösung eingeleitet, bis diese eine Temperatur von 10° erreicht. Man erhält so eine 1,2-Dichloräthanlösung von [6,8-Dichlor-5-(o-fluorphenyl)-2,3-dihydro-1-methyl-2-oxo-1H-1,4-benzodiazepin-7-yl]isocyanat.

b) Eine eisgekühlte Suspension von 6 g Natriumcarbonat in 6,65 ml Dimethylamin und 40 ml Methylenchlorid gibt man auf einmal zur obigen Lösung des Isocyanats, rührt 24 Stunden bei Raumtemperatur, erhitzt anschließend 2 Stunden unter Rückfluß und engt im Rotationsverdampfer ein. Der Rückstand wird in 1 l Methylenchlorid/Äthanol (4 : 1) aufgenommen, mit gesättigter Kochsalzlösung gewaschen, mit Magnesiumsulfat getrocknet und eingedampft. Der Rückstand wird an 100 g Silicagel mit Essigester als Elutionsmittel chromatographiert, wobei 3-[6,8-Dichlor-5-(o-fluorphenyl)-2,3-dihydro-1-methyl-2-oxo-1H-1,4-benzodiazepin-7-yl]-1,1-dimethylharnstoff resultiert, der nach Umkristallisieren aus Äther bei 145—147° schmilzt.

## Beispiel 5

Eine 1,2-Dichloräthanlösung von [6,8-Dichlor-5-(o-fluorphenyl)-2,3-dihydro-1-methyl-2-oxo-1H-1,4-benzodiazepin-7-yl]isocyanat, erhalten gemäß Angaben in Absatz a) von Beispiel 4 aus 6 g (17,1 mMol) 7-Amino-6,8-dichlor-5-(o-fluorphenyl)-1,3-dihydro-1-methyl-2H-1,4-benzodiazepin-2-on wird mit einer Suspension von 6,11 g Natriumcarbonat in 6,3 ml N-(2-Hydroxyäthyl)piperazin und 40 ml Methylenchlorid versetzt. Das Reaktionsgemisch wird 138 Stunden bei Raumtemperatur gerührt und im Vakuum eingeengt. Der Rückstand wird in 1,5 l Methylenchlorid/Äthanol (4 : 1) aufgenommen; die Lösung mit 300 ml Wasser gewaschen, über Magnesiumsulfat getrocknet und eingedampft. Der Rückstand wird an 250 g Kieselgel mit Chloroform als Elutionsmittel chromatographiert, wobei N-[6,8-Dichlor-5-(o-fluorphenyl)-2,3-dihydro-1-methyl-2-oxo-1H-1,4-benzodiazepin-7-yl]-4-(2-hydroxyäthyl)-1-piperazincarboxamid resultiert. Aus Methylenchlorid/Hexan umkristallisiert, schmilzt das Produkt bei 232—233°.

## Beispiel 6

Eine 1,2-Dichloräthanlösung von [6,8-Dichlor-5-(o-fluorphenyl)-2,3-dihydro-1-methyl-2-oxo-1H-1,4-benzodiazepin-7-yl]isocyanat, erhalten gemäß Angaben in Absatz a) von Beispiel 4 aus 5,5 g (15,62 mMol) 7-Amino-6,8-dichlor-5-(o-fluorphenyl)-1,3-dihydro-1-methyl-2H-1,4-benzodiazepin-2-on, wird bei Raumtemperatur mit einer Suspension von 6,11 g Natriumcarbonat in 6,1 ml N-(2-Aminoäthyl-morpholin und 40 ml Dichloräthan auf einmal versetzt und während 114 Stunden gerührt. Das Reaktionsgemisch wird mit Methylenchlorid/Äthanol (4 : 1) extrahiert, die organische Phase mit Wasser gewaschen, getrocknet und eingedampft. Der Rückstand wird an 250 g Kieselgel unter Eluieren mit Chloroform chromatographiert, wobei 1-[6,8-Dichlor-5-(o-fluorphenyl)-2,3-dihydro-1-methyl-2-oxo-1H-1,4-benzodiazepin-7-yl]-3-(2-morpholinoäthyl)harnstoff resultiert. Nach Umkristallisieren aus Äther schmilzt das Produkt bei 174—176°.

## Beispiel 7

Eine 1,2-Dichloräthanlösung von [6,8-Dichlor-5-(o-fluorphenyl)-2,3-dihydro-1-methyl-2-oxo-1H-1,4-benzodiazepin-7-yl]isocyanat, erhalten gemäß Angaben in Absatz a) von Beispiel 4 aus 5 g (14,2 mMol) 7-Amino-6,8-dichlor-5-(o-fluorphenyl)-1,3-dihydro-1-methyl-2H-1,4-benzodiazepin-2-on, wird mit einer Suspension von 6 g Natriumcarbonat in 34 ml Acetonitril und 2 ml Methylamin versetzt. Das Reaktionsgemisch wird 66 Stunden bei Raumtemperatur gerührt, im Vakuum eingeengt und in 1,5 l Methylenchlorid/Äthanol (4 : 1) aufgenommen. Die organische Phase wird mit gesättigter Natriumchloridlösung gewaschen, getrocknet und eingedampft. Den Rückstand chromatographiert man an 300 g Kieselgel mit Methylenchlorid/Essigester (4 : 1) als Elutionsmittel, wobei 1-[6,8-Dichlor-5-(o-fluorphenyl)-2,3-dihydro-1-methyl-2-oxo-1H-1,4-benzodiazepin-7-yl]-3-methylharnstoff resultiert. Aus Äther umkristallisiert, schmilzt das Produkt bei 213–214°.

## Beispiel 8

a) Zu einer Lösung von 107,2 g (0,48 Mol) Carbobenzoxy-DL-alanin in 800 ml Tetrahydrofuran tropft man unter Rühren und Eiskühlen 40 ml (65,6 g, 0,55 Mol) Thionylchlorid zu, rührt anschließend das Gemisch 1 Stunde bei Raumtemperatur, gibt eine Lösung von 108 g (0,39 Mol) 2-Amino-2′-chlor-5-nitro-benzophenon hinzu, rührt bei Raumtemperatur während 24 Stunden und engt das Reaktionsgemisch im Vakuum ein. Der Rückstand wird mit 600 ml 10%iger Natriumbicarbonatlösung versetzt und mit Methylenchlorid/Äthanol (9 : 1) ausgeschüttelt. Die organische Phase wird mit Wasser gewaschen, getrocknet und eingedampft. Der Rückstand wird sofort als solcher weiterverarbeitet.

b) Das obige Rohprodukt wird mit 660 ml 33%iger Bromwasserstoffsäure in Eisessig versetzt und 4 Stunden bei Raumtemperatur kräftig gerührt. Nach Einengen des Reaktionsgemisches im Vakuum wird der Rückstand zwischen 1,5 l Wasser und 1,5 l Äther verteilt; die abgetrennte wässerige Phase neutralisiert man mit 50 g Kaliumcarbonat, extrahiert mit Methylenchlorid, wäscht die organische Phase mit Wasser, trocknet und dampft sie ein. Der Rückstand wird als solcher weiterverarbeitet.

c) Das unter b) erhaltene Rohprodukt, in 1 l Toluol und 100 ml Essigsäure gelöst, kocht man in einem Wasserabscheider während 4 Stunden am Rückfluß und dampft anschließend die Lösung im Vakuum zur Trockne ein. Verbleibende Essigsäure entfernt man durch zweimaliges azeotropes Eindampfen mit Toluol. Der Rückstand, rac-5-(o-Chlorphenyl)-1,3-dihydro-3-methyl-7-nitro-2H-1,4-benzodiazepin-2-on, wird als solcher weiterverarbeitet.

d) 105,7 g (0,32 Mol) rohes rac-5-(o-Chlorphenyl-1,3-dihydro-3-methyl-7-nitro-2H-1,4-benzodiazepin-2-on, in 1 l Aceton gelöst, versetzt man mit 88,6 g (0,64 Mol) Kaliumcarbonat und 39,9 ml (0,64 Mol) Methyljodid und rührt das Gemisch 22 Stunden bei Raumtemperatur. Das Reaktionsgemisch wird zur Trockne eingedampft und der Rückstand in Methylenchlorid aufgenommen. Die organische Phase wird mit Wasser gewaschen, getrocknet und eingedampft. Aus Methylenchlorid/Äthanol resultiert rac-5-(o-Chlorphenyl)-1,3-dihydro-1,3-dimethyl-7-nitro-2H-1,4-benzodiazepin-2-on vom Smp. 170–174°.

e) 108,9 g (0,316 Mol) rac-5-(o-Chlorphenyl)-1,3-dihydro-1,3-dimethyl-7-nitro-2H-1,4-benzodiazepin-2-on werden in 750 ml konzentrierter Salzsäure gelöst. Dazu gibt man unter Eiskühlen und Rühren insgesamt 215 g (0,95 Mol) Zinnchlorid portionenweise zu und rührt das Gemisch 3 Stunden bei Raumtemperatur. Bei 0° neutralisiert man anschließend das Reaktionsgemisch mit 10 N-Natronlauge und extrahiert die wässerige Phase mit Methylenchlorid/Äthanol (4 : 1). Die organische Phase wird mit Wasser gewaschen, getrocknet und eingedampft. Aus Essigester/Petroläther resultiert rac-7-Amino-5-(o-chlorphenyl)-1,3-dihydro-1,3-dimethyl-2H-1,4-benzodiazepin-2-on vom Smp. 180°.

f) Zu einer bei Raumtemperatur gerührten Lösung von 30 g (0,095 Mol) rac-7-Amino-5-(o-chlorphenyl)-1,3-dihydro-1,3-dimethyl-2H-1,4-benzodiazepin-2-on in 500 ml Methylenchlorid gibt man 26,8 g (0,2 Mol) N-Chlorsuccinimid und rührt während 65 Stunden. Man verdünnt anschließend mit 400 ml Methylenchlorid, gibt 500 ml 2N Natriumcarbonatlösung zu, trennt die Phasen und wäscht die organische Phase mit Wasser. Nach dem Trocknen und Einengen des Lösungsmittels im Vakuum chromatographiert man den Rückstand an 1 kg Kieselgel mit Chloroform als Elutionsmittel. Aus Methylenchlorid/Cyclohexan resusltiert rac-7-Amino-6,8-dichlor-5-(o-chlorphenyl)-1,3-dihydro-1,3-dimethyl-2H-1,4-benzodiazepin-2-on vom Smp. 186–187°.

## Beispiel A

1-[6,8-Dichlor-5-(o-fluorphenyl)-2,3-dihydro-1-methyl-2-oxo-1H-1,4-benzodiazepin-7-yl]-3-(2-morpholinoäthyl)harnstoff kann wie folgt als Wirkstoff zur Herstellung von pharmazeutischen Präparaten verwendet werden:

a) Tabletten

| | |
|---|---|
| 1 Tablette enthält: | |
| Wirkstoff | 200 mg |
| Mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10%igen Lösung von Hydroxypropylmethylcellulose in einem Gemisch von Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpreßt.

b) Kapseln

| | |
|---|---|
| 1 Kapsel enthält: | |
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Größe abgefüllt.

**Patentansprüche**

1. Benzodiazepin-Derivate der allgemeinen Formel

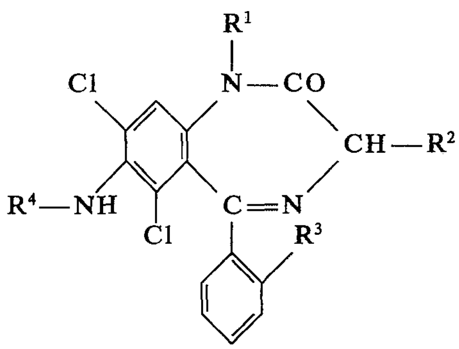

(I)

worin $R^1$ $C_{1-7}$-Alkyl, $R^2$ Wasserstoff oder $C_{1-7}$-Alkyl, $R^3$ Wasserstoff oder Halogen, $R^4$ Wasserstoff oder einen Rest der Formel

$R^5—CO—$

$R^6R^7N—CO—$

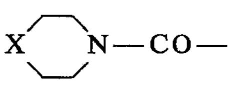

oder

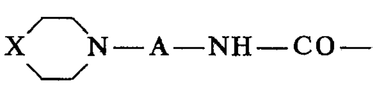

A $C_{1-7}$-Alkylen, X ein Sauerstoff- oder Schwefelatom oder einen Rest der Formel $> N-R^8$, $R^5$

$C_{1-7}$-Alkyl oder $C_{1-7}$-Haloalkyl, $R^6$ Wasserstoff oder $C_{1-7}$-Alkyl, $R^7$ $C_{1-7}$-Alkyl und $R^8$ Wasserstoff, $C_{1-7}$-Alkyl oder $C_{2-7}$-Hydroxyalkyl bedeuten,
und pharmazeutisch akzeptable Säureadditionssalze davon.

2. Verbindungen gemäß Anspruch 1, dadurch gekennzeichnet, daß $R^1$ Methyl bedeutet.

3. Verbindungen gemäß Anspruch 1 oder 2, dadurch gekennzeichnet, daß $R^2$ Wasserstoff oder Methyl bedeutet.

4. Verbindungen gemäß einem der Ansprüche 1 bis 3, dadurch gekennzeichnet, daß $R^3$ Wasserstoff, Fluor oder Chlor bedeutet.

5. Verbindungen gemäß einem der Ansprüche 1 bis 4, dadurch gekennzeichnet, daß $R^4$ Wasserstoff oder einen Rest der Formel

$(CH_3)_2N—CO—$

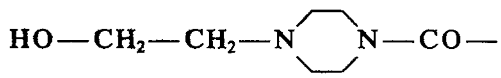

oder

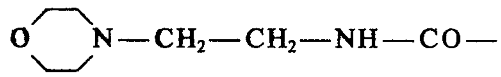

bedeutet.

6. 3-[6,8-Dichlor-5-(o-fluorphenyl)-2,3-dihydro-1-methyl-2-oxo-1H-1,4-benzodiazepin-7-yl]-1,1-dimethylharnstoff.

7. N-[6,8-Dichlor-5-(o-fluorphenyl)-2,3-dihydro-1-methyl-2-oxo-1H-1,4-benzodiazepin-7-yl]-4-(2-hydroxyäthyl)-1-piperazincarboxamid.

8. 1-[6,8-Dichlor-5-(o-fluorphenyl)-2,3-dihydro-1-methyl-2-oxo-1H-1,4-benzodiazepin-7-yl]-3-(2-morpholinoäthyl)harnstoff.

9. 7-Amino-6,8-dichlor-5-phenyl-1,3-dihydro-1-methyl-2H-1,4-benzodiazepin-2-on,
7-Amino-6,8-dichlor-5-(o-chlorphenyl)-1,3-dihydro-1,3-dimethyl-2H-1,4-benzodiazepin-2-on,
3-[6,8-Dichlor-5-(o-fluorphenyl)-2,3-dihydro-1-methyl-2-oxo-1H-1,4-benzodiazepin-7-yl]-1-methylharnstoff,
N-[6,8-Dichlor-5-(o-fluorphenyl)-2,3-dihydro-1-methyl-2-oxo-1H-1,4-benzodiazepin-7-yl]-2,2,2-trifluoracetamid und
7-Amino-6,8-dichlor-5-(o-fluorphenyl)-1,3-dihydro-1-methyl-2H-1,4-benzodiazepin-2-on.

10. Verbindungen der allgemeinen Formel

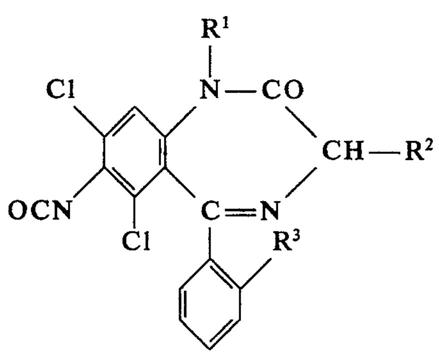

(VII)

worin $R^1$, $R^2$ und $R^3$ die in Anspruch 1 angegebene Bedeutung besitzen.

11. Verbindungen der allgemeinen Formel

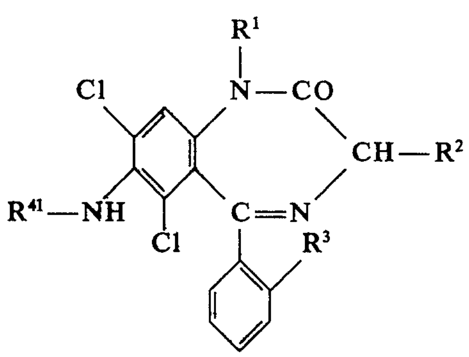

(XI)

worin $R^{41}$ eine Schutzgruppe oder einen Rest der Formel

$R^{62}R^{72}N—CO—$

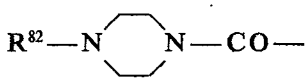

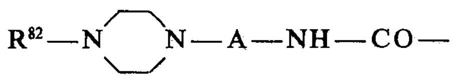

oder

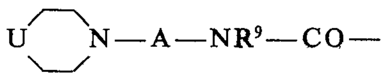

U ein Sauerstoff- oder Schwefelatom oder einen Rest der Formel $>N—R^{83}$, $R^{62}$ eine Schutzgruppe, $R^{72}$ $C_{1-7}$-Alkyl, $R^{82}$ eine Schutzgruppe oder einen Rest der Formel —A—O—V, $R^{83}$ Wasserstoff, $C_{1-7}$-Alkyl, $C_{2-7}$-Hydroxyalkyl, eine Schutzgruppe oder einen Rest der Formel —A—O—V und $R^9$ und V je eine Schutzgruppe bedeuten und $R^1$, $R^2$, $R^3$ und A die in Anspruch 1 angegebene Bedeutung besitzen.

12. Verbindungen der allgemeinen Formel

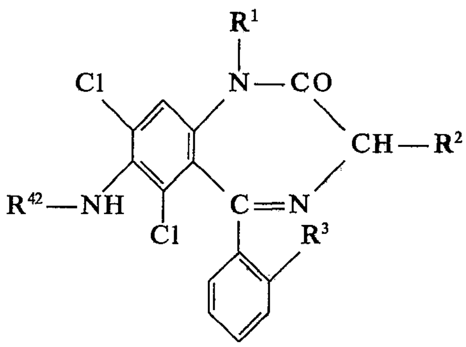

(XII)

worin $R^{42}$ einen Rest der Formel

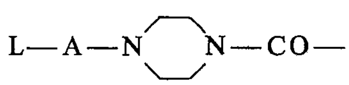

oder

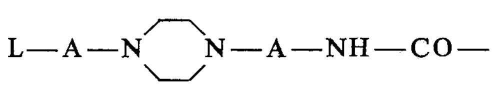

und L eine Abgangsgruppe bedeuten, und $R^1$, $R^2$, $R^3$ und A die in Anspruch 1 angegebene Bedeutung besitzen,

13. Verbindungen der allgemeinen Formel

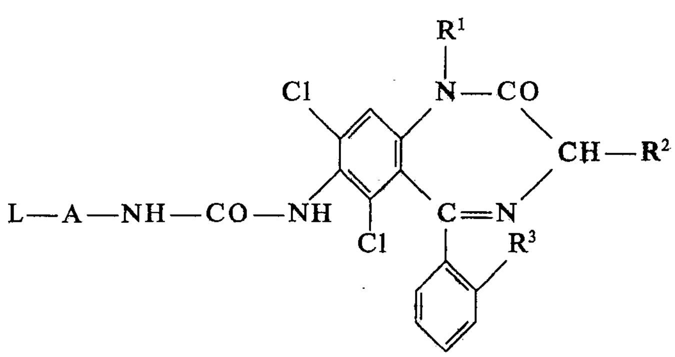

(XIII)

worin $R^1$, $R^2$, $R^3$ und A die in Anspruch 1 angegebene Bedeutung besitzen und L eine Abgangsgruppe bedeutet.

14. Verbindungen der allgemeinen Formel

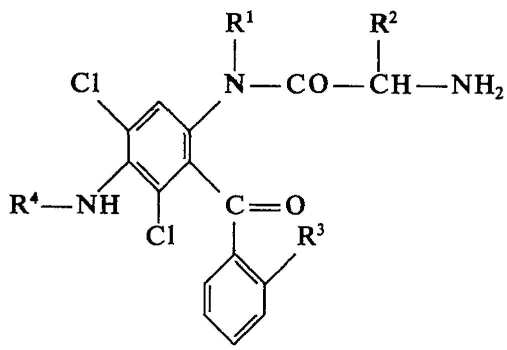

(XIV)

worin $R^1$, $R^2$, $R^3$ und $R^4$ die in Anspruch 1 angegebene Bedeutung besitzen.

15. Verbindungen gemäß einem der Ansprüche 1 bis 9 und pharmazeutische akzeptable Säureadditionssalze davon als pharmazeutische Wirkstoffe.

16. Verbindungen gemäß einem der Ansprüche 1 bis 9 und pharmazeutisch akzeptable Säureadditionssalze davon als aldosteronantagonistische Wirkstoffe.

17. Verfahren zur Herstellung von Verbindungen gemäß einem der Ansprüche 1 bis 9, dadurch gekennzeichnet, daß man

a) ein Benzodiazepin-Derivat der allgemeinen Formel

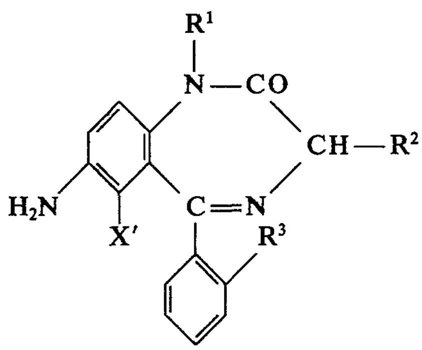

(II)

worin $R^1$, $R^2$ und $R^3$ die in Anspruch 1 angegebene Bedeutung besitzen, und X' Wasserstoff oder Chlor bedeutet,
chloriert, oder

b) ein Benzodiazepin-Derivat der allgemeinen Formel

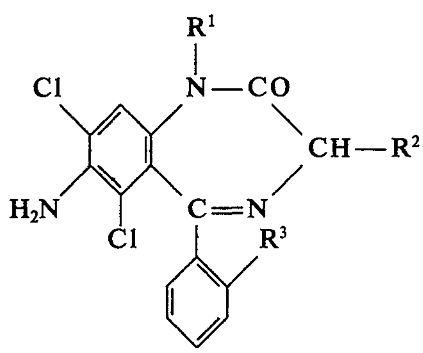

(Ia)

worin $R^1$, $R^2$ und $R^3$ die in Anspruch 1 angegebene Bedeutung besitzen,
mit einer Säure der Formel $R^5$—COOH, worin $R^5$ die in Anspruch 1 angegebene Bedeutung besitzt, oder einem reaktiven Derivat davon acyliert, oder

c) ein Benzodiazepin-Derivat der obigen allgemeinen Formel Ia mit einem Halogenid der allgemeinen Formel

$R^{61}R^{71}N-CO-Y$ (III)

oder

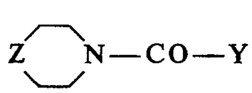

(IV)

worin Y Halogen, $R^{61}$ und $R^{71}$ je $C_{1-7}$-Alkyl, Z ein Sauerstoff- oder Schwefelatom oder einen Rest der Formel >N—$R^{81}$ und $R^{81}$ $C_{1-7}$-Alkyl bedeuten,
umsetzt, oder

d) ein Benzodiazepin-Derivat der obigen allgemeinen Formel Ia mit einem Isocyanat der allgemeinen Formel

$R^{71}NCO$ (V)

oder

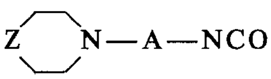 (VI)

worin A die in Anspruch 1 angegebene und Z und $R^{71}$ obige Bedeutung besitzen,
umsetzt, oder

e) ein Benzodiazepin-Derivat der allgemeinen Formel

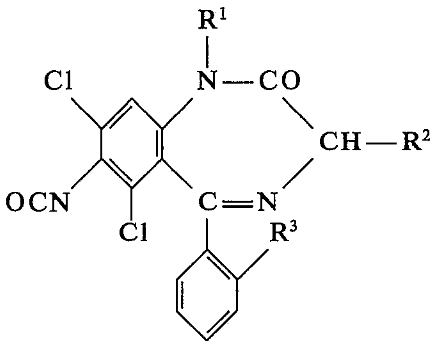 (VII)

worin $R^1$, $R^2$ und $R^3$ die in Anspruch 1 angegebene Bedeutung besitzen,
mit einer Aminoverbindung der allgemeinen Formel

$R^6R^7NH$ (VIII)

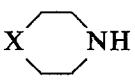 (IX)

oder

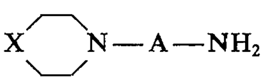 (X)

worin A, X, $R^6$ und $R^7$ die in Anspruch 1 angegebene Bedeutung besitzen,
umsetzt, oder

f) aus einem Benzodiazepin-Derivat der allgemeinen Formel

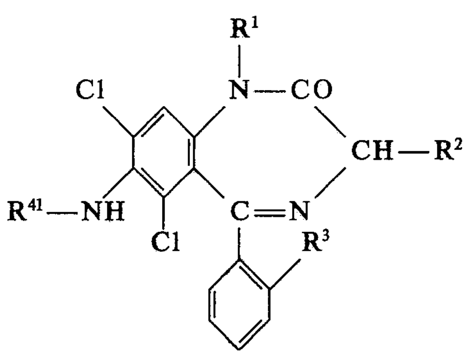 (XI)

worin $R^{41}$ eine Schutzgruppe oder einen Rest der Formel

$R^{62}R^{72}N—CO—$

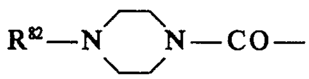

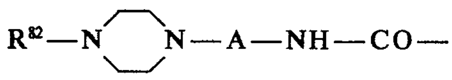

oder

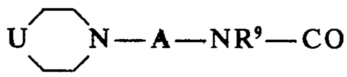

U ein Sauerstoff- oder Schwefelatom oder einen Rest der Formel $>N-R^{83}$, $R^{62}$ eine Schutzgruppe, $R^{72}$ $C_{1-7}$-Alkyl, $R^{82}$ eine Schutzgruppe oder einen Rest der Formel –A–O–V, $R^{83}$ Wasserstoff, $C_{1-7}$-Alkyl, $C_{2-7}$-Hydroxyalkyl, eine Schutzgruppe oder einen Rest der Formel –A–O–V, $R^9$ und V eine Schutzgruppe bedeuten und $R^1$, $R^2$, $R^3$ und A die in Anspruch 1 angegebene Bedeutung besitzen,

die Schutzgruppe(n) entfernt, oder

g) ein Benzodiazepin-Derivat der allgemeinen Formel

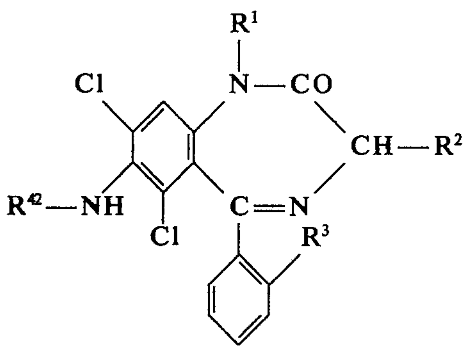

(XII)

worin $R^{42}$ einen Rest der Formel

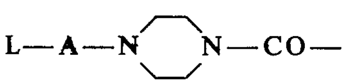

oder

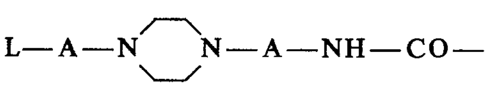

und L eine Abgangsgruppe bedeuten, und $R^1$, $R^2$, $R^3$ und A die in Anspruch 1 angegebene Bedeutung besitzen,

in die entsprechende Hydroxyverbindung überführt, oder

h) ein Benzodiazepin-Derivat der allgemeinen Formel

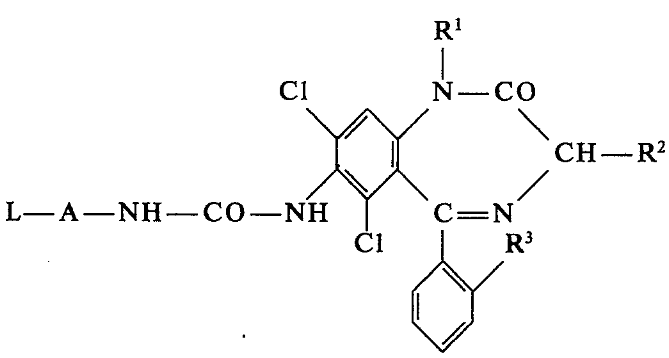

(XIII)

worin $R^1$, $R^2$, $R^3$ und A die in Anspruch 1 angegebene und L obige Bedeutung besitzen, mit einer Verbindung der Formel

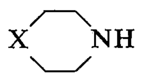

worin X die in Anspruch 1 angegebene Bedeutung besitzt, umsetzt, oder

i) ein Benzodiazepin-Derivat der allgemeinen Formel

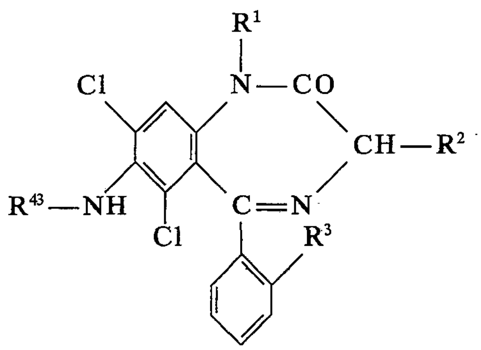

(Ib)

worin $R^{43}$ einen Rest der Formel

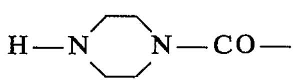

oder

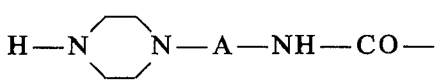

$R^1$, $R^2$, $R^3$ und A die in Anspruch 1 angegebene Bedeutung besitzen, alkyliert, oder

k) ein Benzophenon-Derivat der allgemeinen Formel

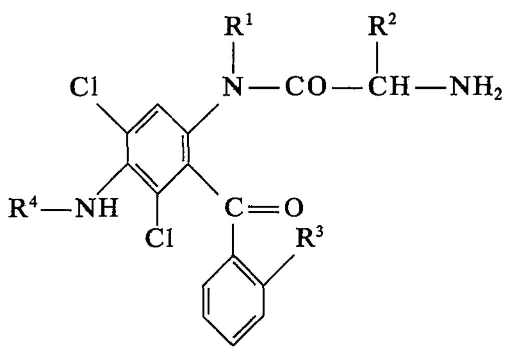

(XIV)

worin $R^1$, $R^2$, $R^3$ und $R^4$ die in Anspruch 1 angegebene Bedeutung besitzen, cyclisiert, oder

l) ein Benzodiazepin-Derivat der allgemeinen Formel I in ein pharmazeutisch akzeptables Säureadditionssalz überführt.

18. Arzneimittel, enthaltend eine Verbindung gemäß einem der Ansprüche 1 bis 9 oder ein pharmazeutisch akzeptables Säureadditionssalz davon.

19. Aldosteron-antagonistische Arzneimittel, enthaltend eine Verbindung gemäß einem der Ansprüche 1 bis 9 oder ein pharmazeutisch akzeptables Säureadditionssalz davon.

## Claims

1. Benzodiazepine derivatives of the general formula

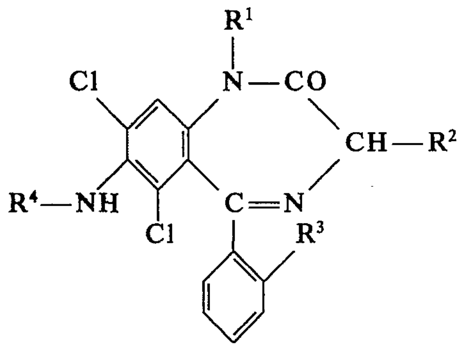

(I)

wherein $R^1$ signifies $C_{1-7}$-alkyl, $R^2$ signifies hydrogen or $C_{1-7}$-alkyl, $R^3$ signifies hydrogen or halogen, $R^4$ signifies hydrogen or a residue of the formula

$R^5—CO—$

$R^6R^7N—CO—$

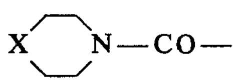

or

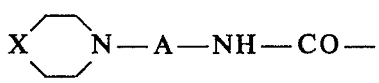

A signifies $C_{1-7}$-alkylene, X signifies an oxygen or sulphur atom or a residue of the formula $> N-R^8$, $R^5$ signifies $C_{1-7}$-alkyl or $C_{1-7}$-haloalkyl, $R^6$ signifies hydrogen or $C_{1-7}$-alkyl, $R^7$ signifies $C_{1-7}$-alkyl and $R^8$ signifies hydrogen, $C_{1-7}$-alkyl or $C_{2-7}$-hydroxyalkyl,
and pharmaceutically acceptable acid addition salts thereof.

2. Compounds according to claim 1, characterized in that $R^1$ signifies methyl.

3. Compounds according to claim 1 or 2, characterized in that $R^2$ signifies hydrogen or methyl.

4. Compounds according to any one of claims 1 to 3, characterized in that $R^3$ signifies hydrogen, fluorine or chlorine.

5. Compounds according to any one of claims 1 to 4, characterized in that $R^4$ signifies hydrogen or a residue of the formula

$(CH_3)_2N—CO—$

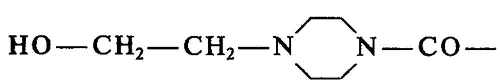

or

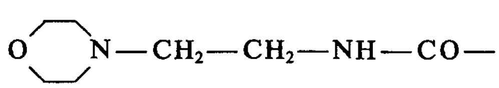

6. 3-[6,8-Dichloro-5-(o-fluorophenyl)-2,3-dihydro-1-methyl-2-oxo-1H-1,4-benzodiazepin-7-yl]-1,1-dimethylurea.

7. N-[6,8-Dichloro-5-(o-fluorophenyl)-2,3-dihydro-1-methyl-2-oxo-1H-1,4-benzodiazepin-7-yl]-4-(2-hydroxyethyl)-1-piperazinecarboxamide.

8. 1-[6,8-Dichloro-5-(o-fluorophenyl)-2,3-dihydro-1-methyl-2-oxo-1H-1,4-benzodiazepin-7-yl]-3-(2-morpholinoethyl)urea.

9. 7-Amino-6,8-dichloro-5-phenyl-1,3-dihydro-1-methyl-2H-1,4-benzodiazepin-2-one,
7-amino-6,8-dichloro-5-(o-chlorophenyl)-1,3-dihydro-1,3-dimethyl-2H-1,4-benzodiazepin-2-one,
3-[6,8-dichloro-5-(o-fluorophenyl)-2,3-dihydro-1-methyl-2-oxo-1H-1,4-benzodiazepin-7-yl]-1-methylurea,

N-[6,8-dichloro-5-(o-fluorophenyl)-2,3-dihydro-1-methyl-2-oxo-1H-1,4-benzodiazepin-7-yl]-2,2,2-trifluoroacetamide and
7-amino-6,8-dichloro-5-(o-fluorophenyl)-1,3-dihydro-1-methyl-2H-1,4-benzodiazepin-2-one.

10. Compounds of the general formula

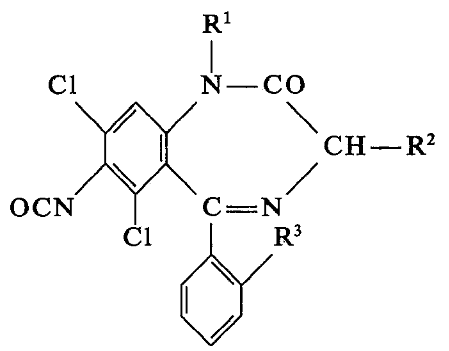

(VII)

wherein $R^1$, $R^2$ and $R^3$ have the significance given in claim 1.

11. Compounds of the general formula

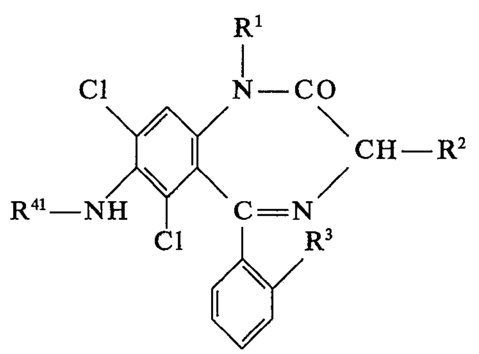

(XI)

wherein $R^{41}$ signifies a protecting group or a residue of the formula

$R^{62}R^{72}N—CO—$

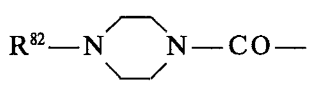

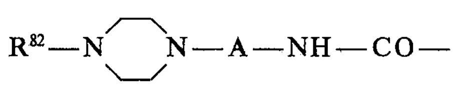

or

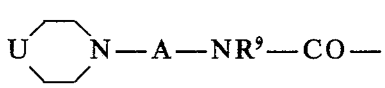

U signifies an oxygen or sulphur atom or a residue of the formula $>N–R^{83}$, $R^{62}$ signifies a protecting group, $R^{72}$ signifies $C_{1-7}$-alkyl, $R^{82}$ signifies a protecting group or a residue of the formula –A–O–V, $R^{83}$ signifies hydrogen, $C_{1-7}$-alkyl, $C_{2-7}$-hydroxyalkyl, a protecting group or a residue of the formula –A–O–V and $R^9$ and V each signify a protecting group and $R^1$, $R^2$, $R^3$ and A have the significance given in claim 1.

12. Compounds of the general formula

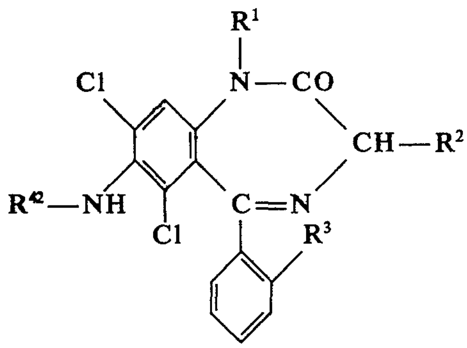

(XII)

wherein $R^{42}$ signifies a residue of the formula

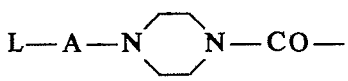

or

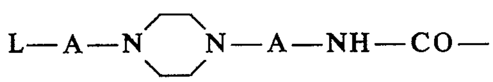

and L signifies a leaving group, and $R^1$, $R^2$, $R^3$ and A have the significance given in claim 1.

13. Compounds of the general formula

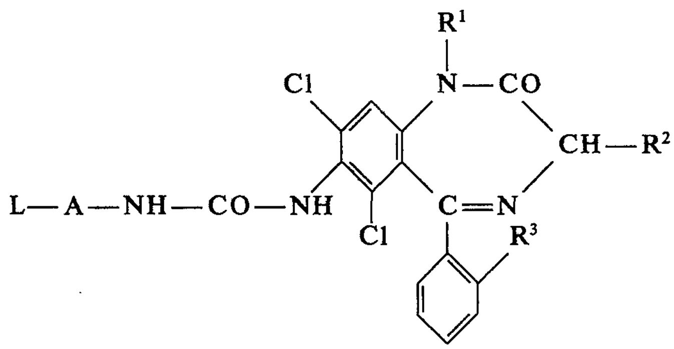

(XIII)

wherein $R^1$, $R^2$, $R^3$ and A have the significance given in claim 1 and L signifies a leaving group.

14. Compounds of the general formula

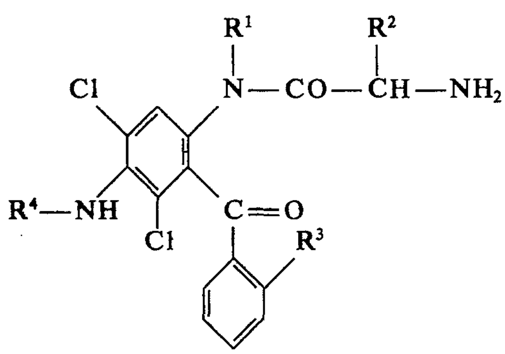

(XIV)

wherein $R^1$, $R^2$, $R^3$ and $R^4$ have the significance given in claim 1.

15. Compounds according to any one of claims 1 to 9 and pharmaceutically acceptable acid addition salts thereof as pharmaceutically active substances.

16. Compounds according to any one of claims 1 to 9 and pharmaceutically acceptable acid addition salts thereof as aldosterone-antagonistic active substances.

17. Process for the manufacture of compounds according to any one of claims 1 to 9, characterized by

a) chlorinating a benzodiazepine derivative of the general formula

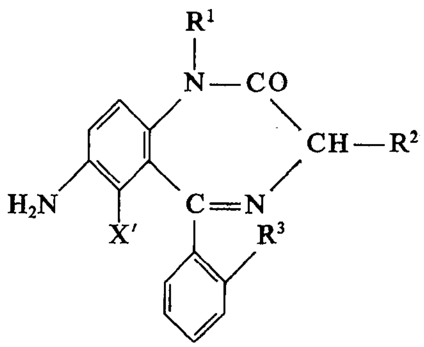

(II)

wherein $R^1$, $R^2$ and $R^3$ have the significance given in claim 1, and X' signifies hydrogen or chlorine, or

b) acylating a benzodiazepine derivative of the general formula

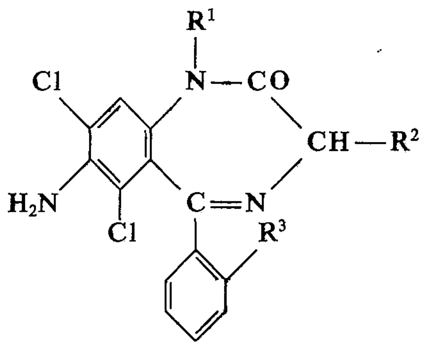

(Ia)

wherein $R^1$, $R^2$ and $R^3$ have the significance given in claim 1, with an acid of the formula $R^5$—COOH, wherein $R^5$ has the significance given in claim 1, or a reactive derivative thereof, or

c) reacting an benzodiazepine derivative of general formula Ia above with a halide of the general formula

$R^{61}R^{71}N—CO—Y$ (III)

or

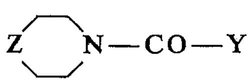

(IV)

wherein Y signifies halogen, $R^{61}$ and $R^{71}$ each signify $C_{1-7}$-alkyl, Z signifies an oxygen or sulphur atom or a residue of the formula $>$N—$R^{81}$ and $R^{81}$ signifies $C_{1-7}$-alkyl, or

d) reacting a benzodiazepine derivative of general formula Ia above with an isocyanate of the general formula

$R^{71}NCO$ (V)

or

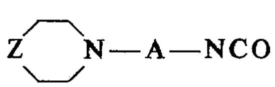

(VI)

wherein A has the significance given in claim 1 and Z and $R^{71}$ have the above significance, or

e) reacting a benzodiazepine derivative of the general formula

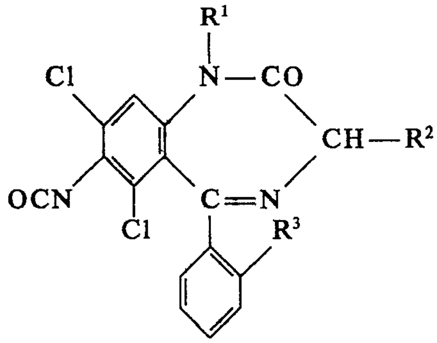

(VII)

wherein $R^1$, $R^2$ and $R^3$ have the significance given in claim 1, with an amino compound of the general formula

$R^6R^7NH$ (VIII)

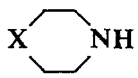

(IX)

or

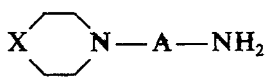

(X)

wherein A, X, $R^6$ and $R^7$ have the significance given in claim 1,
or

f) removing the protecting group(s) from a benzodiazepine derivative of the general formula

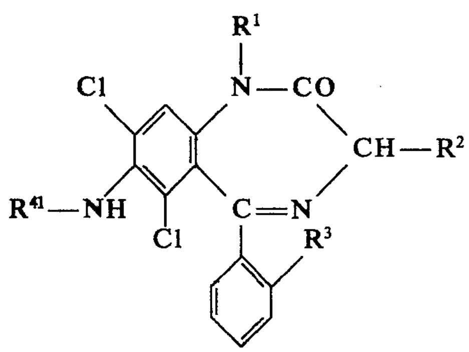

(XI)

wherein $R^{41}$ signifies a protecting group or a residue of the formula

$R^{62}R^{72}N—CO—$

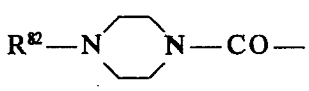

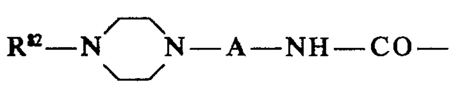

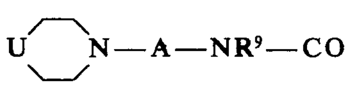

U signifies an oxygen or sulphur atom or a residue of the formula $>N—R^{83}$, $R^{62}$ signifies a protecting group, $R^{72}$ signifies $C_{1-7}$-alkyl, $R^{82}$ signifies a protecting group or a residue of the formula —A—O—V, $R^{83}$ signifies hydrogen, $C_{1-7}$-alkyl, $C_{2-7}$-hydroxyalkyl, a protecting group or a residue of the formula —A—O—V, $R^9$ and V signify a protecting group, and $R^1$, $R^2$, $R^3$ and A have the significance given in claim 1,
or

g) converting a benzodiazepine derivative of the general formula

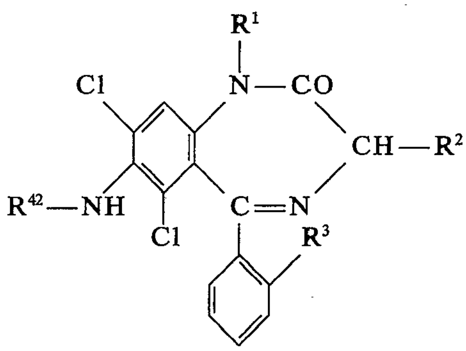

(XII)

wherein $R^{42}$ signifies a residue of the formula

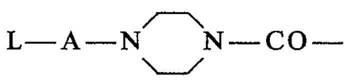

or

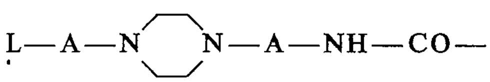

and L signifies a leaving group, and $R^1$, $R^2$, $R^3$ and A have the significance given in claim 1, into the corresponding hydroxy compound, or

h) reacting a benzodiazepine derivative of the general formula

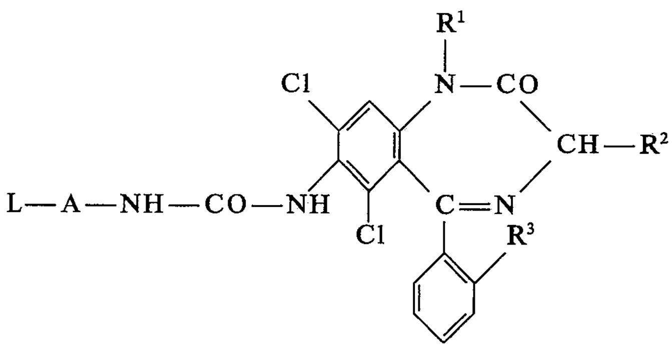

(XIII)

wherein $R^1$, $R^2$, $R^3$ and A have the significance given in claim 1 and L has the above significance, with a compound of the formula

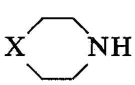

wherein X has the significance given in claim 1,
or

i) alkylating a benzodiazepine derivative of the general formula

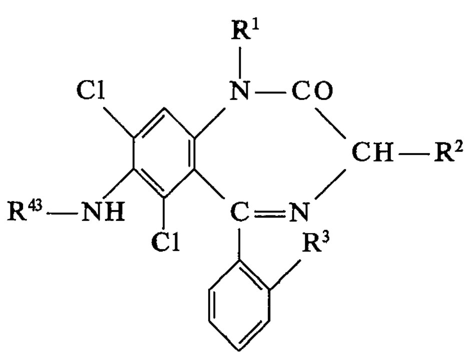

(Ib)

wherein $R^{43}$ signifies a residue of the formula

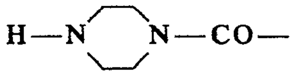

or

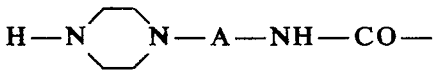

$R^1$, $R^2$, $R^3$ and A have the significance given in claim 1,
or

k) cyclizing a benzophenone derivative of the general formula

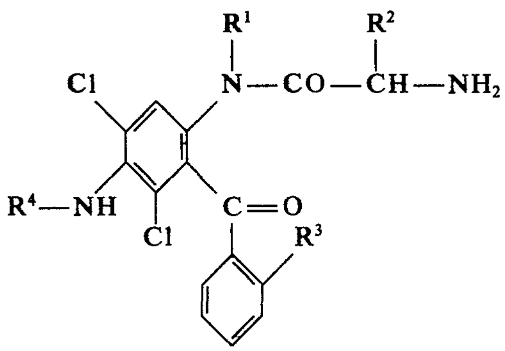

(XIV)

wherein $R^1$, $R^2$, $R^3$ and $R^4$ have the significance given in claim 1,
or

l) converting a benzodiazepine derivative of general formula I into a pharmaceuticall acceptable acid addition salt.

18. Medicament, containing a compound according to any one of claims 1 to 9 or a pharmaceutically acceptable acid addition salt thereof.

19. Aldosterone-antagonistic medicament, containing a compound according to any one of claims 1 to 9 or a pharmaceutically acceptable acid addition salt thereof.

**Revendications**

1. Dérivés de benzodiazépine de formule générale

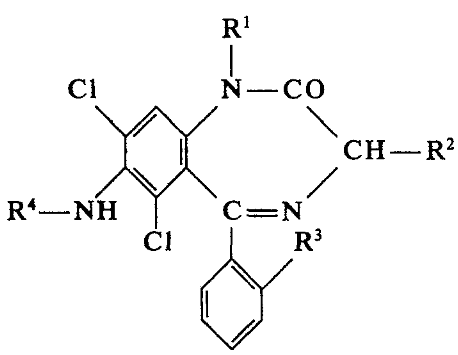

(I)

où $R^1$ représente un alcoyle en $C_1$ à $C_7$, $R^2$ représente un hydrogène ou un alcoyle en $C_1$ à $C_7$, $R^3$ représente un hydrogène ou un halogène, $R^4$ représente un hydrogène ou un radical de formule

$R^5—CO—$

$R^6R^7N—CO—$

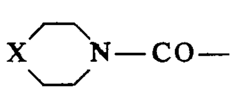

ou

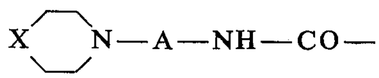

A représente un alcoylène en $C_1$ à $C_7$, X représente un atome d'oxygène ou de soufre ou un radical de formule $>N-R^8$, $R^5$ représente un alcoyle en $C_1$ à $C_7$ ou un haloalcoyle en $C_1$ à $C_7$, $R^6$ représente un hydrogène ou un alcoyle en $C_1$ à $C_7$, $R^7$ représente un alcoyle en $C_1$ à $C_7$ et $R^8$ un hydrogène, un alcoyle ou un hydroxyalcoyle en $C_2$ à $C_7$,
et leurs sels d'addition d'acides pharmaceutiquement acceptables.

2. Composés selon la revendication 1, caractérisés en ce que $R^1$ représente un méthyle.

3. Composés selon l'une des revendications 1 ou 2, caractérisés en ce que $R^2$ représente un hydrogène ou un méthyle.

4. Composés selon l'une des revendications 1 à 3, caractérisés en ce que $R^3$ représente un hydrogène, un fluor ou un chlore.

5. Composés selon l'une des revendications 1 à 4, caractérisés en ce que $R^4$ représente un hydrogène ou un radical de formule

$(CH_3)_2N-CO-$

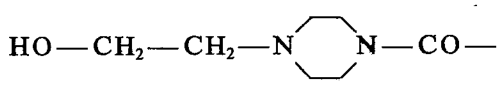

ou

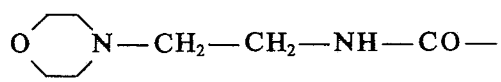

6. 3-[6,8-dichloro-5-(o-fluorophényl)-2,3-dihydro-1-méthyl-2-oxo-1H-1,4-benzodiazépin-7-yl]-1,1-diméthyl-urée.

7. N-[6,8-dichloro-5-(o-fluorophényl)-2,3-dihydro-1-méthyl-2-oxo-1H-1,4-benzodiazépin-7-yl]-4-(2-hydroxyéthyl)-1-pipérazinecarboxamide.

8. 1-[6,8-dichloro-5-(o-fluorophényl)-2,3-dihydro-1-méthyl-2-oxo-1H-1,4-benzodiazépin-7-yl]-3-(2-morpholinoéthyl)urée.

9. 7-amino-6,8-dichloro-5-phényl-1,3-dihydro-1-méthyl-2H-1,4-benzodiazépin-2-one,
7-amino-6,8-dichloro-5-(o-chlorophényl)-1,3-dihydro-1,3-diméthyl-2H-1,4-benzodiazépin-2-one,
3-[6,8-dichloro-5-(o-fluorophényl)-2,3-dihydro-1-méthyl-2-oxo-1H-1,4-benzodiazépin-7-yl]-1-méthylurée,
N-[6,8-dichloro-5-(o-fluorophényl)-2,3-dihydro-1-méthyl-2-oxo-1H-1,4-benzodiazépin-7-yl]-2,2,2-trifluoroacétamide, et
7-amino-6,8-dichloro-5-(o-fluorophényl)-1,3-dihydro-1-méthyl-2H-1,4-benzodiazépin-2-one.

10. Composés de formule générale

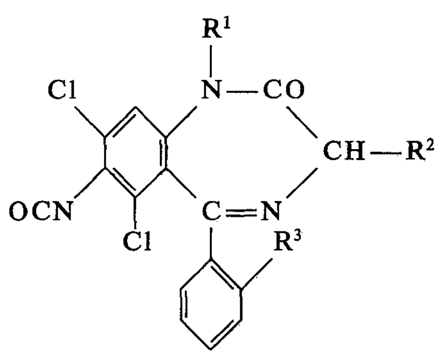

(VII)

où $R^1$, $R^2$ et $R^3$ ont la signification donnée dans la revendication 1.

11. Composés de formule générale

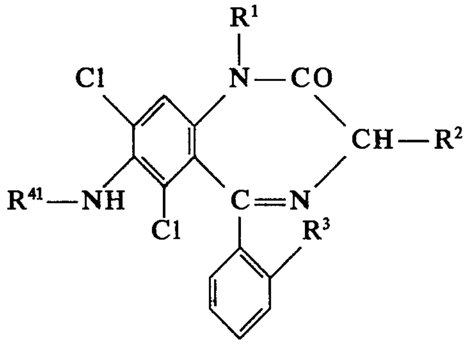

(XI)

où $R^{41}$ représente un groupe protecteur ou un radical de formule

$R^{62}R^{72}N—CO—$

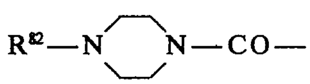

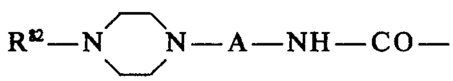

ou

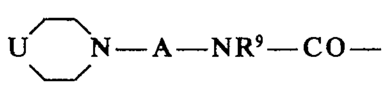

U représente un atome d'oxygène ou de soufre ou un radical de formule $>N—R^{83}$, $R^{62}$ représente un groupe protecteur, $R^{72}$ représente un alcoyle en $C_1$ à $C_7$, $R^{82}$ représente un groupe protecteur ou un radical de formule –A–O–V, $R^{83}$ représente un hydrogène, un alcoyle en $C_1$ à $C_7$, un hydroxyalcoyle en $C_2$ à $C_7$, un groupe protecteur ou un radical de formule –A–O–V, et $R^9$ et V représentent chacun un groupe protecteur et $R^1$, $R^2$, $R^3$ et A ont la signification donnée dans la revendication 1.

12. Composés de formule générale

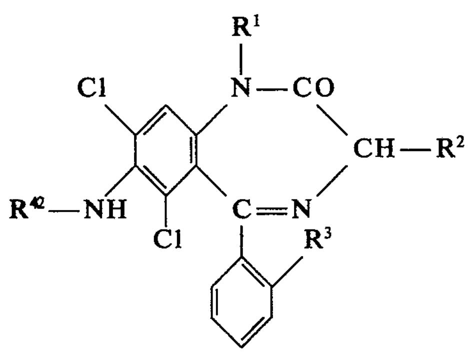

(XII)

où $R^{42}$ représente un radical de formule

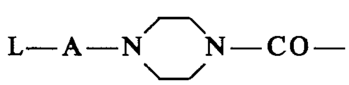

ou

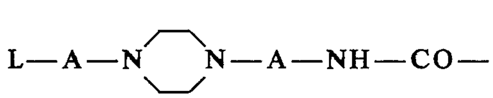

et L représente un groupe sortant, et $R^1$, $R^2$, $R^3$ et A ont la signification donnée dans la revendication 1.

13. Composés de formule générale

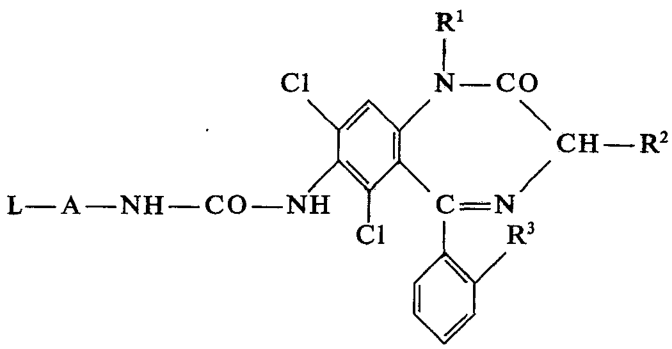

(XIII)

où $R^1$, $R^2$, $R^3$ et A ont la signification donnée dans la revendication 1 et L représente un groupe sortant.

14. Composés de formule générale

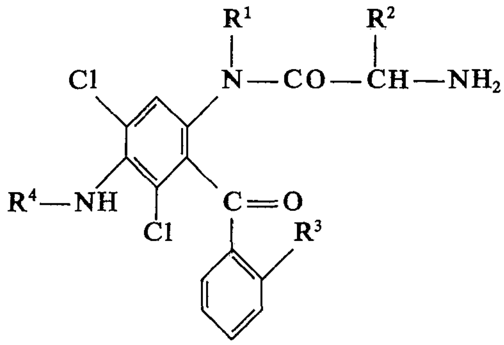

(XIV)

où $R^1$, $R^2$, $R^3$ et $R^4$ ont la signification donnée dans la revendication 1.

15. Composés selon l'une des revendications 1 à 9 et leurs sels d'addition d'acides pharmaceutiquement acceptables comme substances actives pharmaceutiques.

16. Composés selon l'une des revendications 1 à 9 et leurs sels d'addition d'acides pharmaceutiquement acceptables comme substances actives antagonistes de l'aldostérone.

17. Procédé de préparation de composés selon l'une des revendications 1 à 9, caractérisé en ce que

a) on chlore un dérivé de benzodiazépine de formule générale

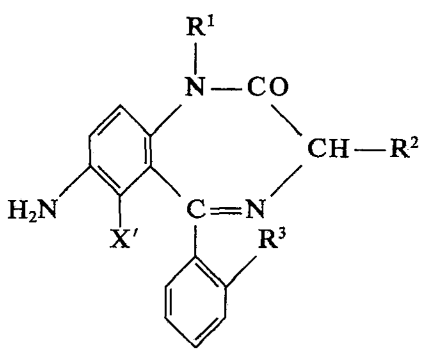

(II)

où $R^1$, $R^2$ et $R^3$ ont la signification donnée dans la revendication 1, et X' représente un hydrogène ou un chlore, ou

b) on acyle un dérivé de benzodiazépine de formule générale

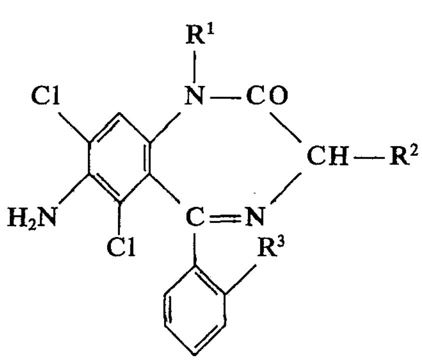

(Ia)

où $R^1$, $R^2$ et $R^3$ ont la signification donnée dans la revendication 1, avec un acide de formule $R^5$—COOH, où $R^5$ a la signification donnée dans la revendication 1, ou avec un de ses dérivés réactifs, ou

c) on fait réagir un dérivé de benzodiazépine de formule générale Ia ci-dessus avec un halogénure de formule générale

$R^{61}R^{71}N—CO—Y$ (III)

ou

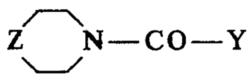

(IV)

où Y représente un halogène, $R^{61}$ et $R^{71}$ chacun un alcoyle en $C_1$ à $C_7$, Z un atome d'oxygène ou de soufre ou un radical de formule >N—$R^{81}$ et $R^{81}$ un alcoyle en $C_1$ à $C_7$, ou

d) on fait réagir un dérivé de benzodiazépine de formule générale Ia ci-dessus avec un isocyanate de formule générale

$R^{71}NCO$ (V)

ou

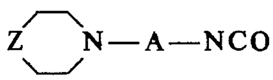

(VI)

où A a la signification donnée dans la revendication 1 et où Z et $R^{71}$ ont la signification donnée ci-dessus, ou

e) on fait réagir un dérivé de benzodiazépine de formule générale

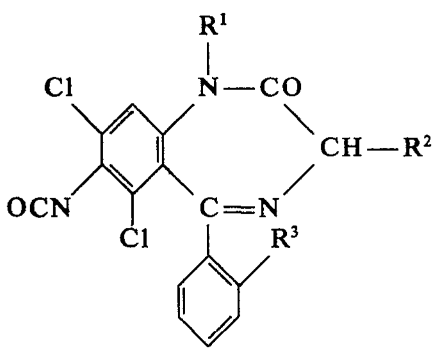

(VII)

où $R^1$, $R^2$ et $R^3$ ont la signification donnée dans la revendication 1, avec un composé amino de formule générale

$R^6R^7NH$ (VIII)

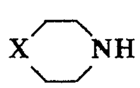

(IX)

ou

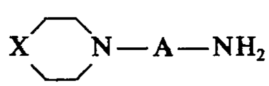

(X)

où A, X, $R^6$ et $R^7$ ont la signification donnée dans la revendication 1, ou

f) on retire le(s) groupe(s) protecteur(s) d'un dérivé de benzodiazépine de formule générale

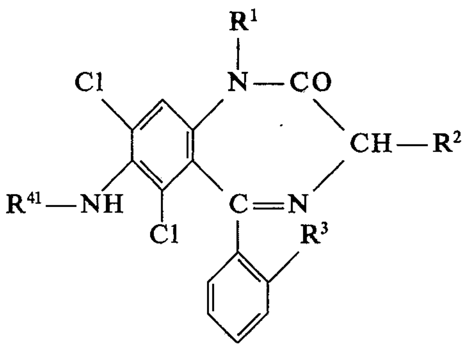

(XI)

où $R^{41}$ représente un groupe protecteur ou un radical de formule

$R^{62}R^{72}N—CO—$

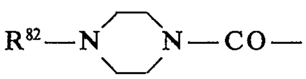

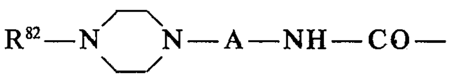

ou

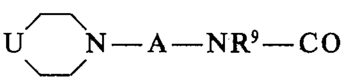

U représente un atome d'oxygène ou de soufre ou un radical de formule $>N—R^{83}$, $R^{62}$ représente un groupe protecteur, $R^{72}$ un alcoyle en $C_1$ à $C_7$, $R^{82}$ un groupe protecteur ou un radical de formule —A—O—V, $R^{83}$ représente un hydrogène, un alcoyle en $C_1$ à $C_7$, un hydroxyalcoyle en $C_2$ à $C_7$, un groupe protecteur ou un radical de formule—A—O—V, et $R^9$ et V représentent un groupe protecteur, et $R^1$, $R^2$, $R^3$ et A ont la signification donnée dans la revendication 1, ou

g) on transforme un dérivé de benzodiazépine der formule générale

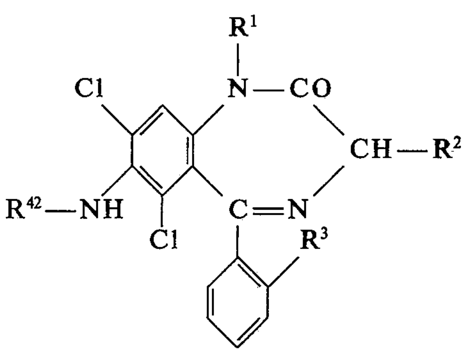

(XII)

où $R^{42}$ représente un radical de formule

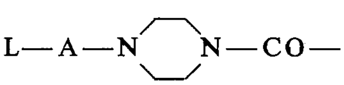

ou

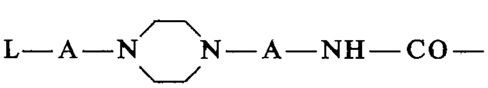

et L représente un groupe sortant, et $R^1$, $R^2$, $R^3$ et A ont la signification donnée dans la revendication 1, en le composé hydroxy correspondant, ou

h) on fait réagir un dérivé de benzodiazépine de formule générale

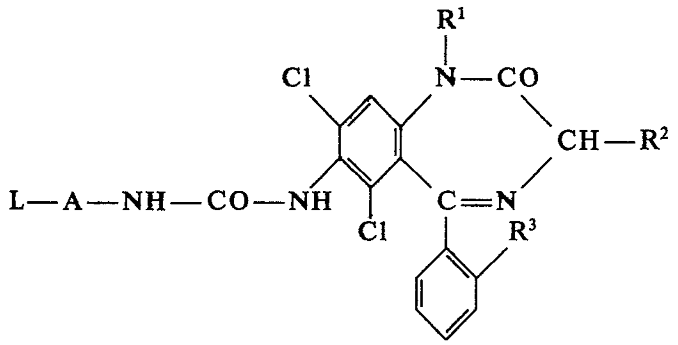

(XIII)

où $R^1$, $R^2$, $R^3$ et A ont la signification donnée dans la revendication 1 et L a la signification donnée ci-desseus, avec un composé de formule

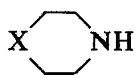

où X a la signification donnée dans la revendication 1, ou

i) on alcoyle un dérivé de benzodiazépine de formule générale

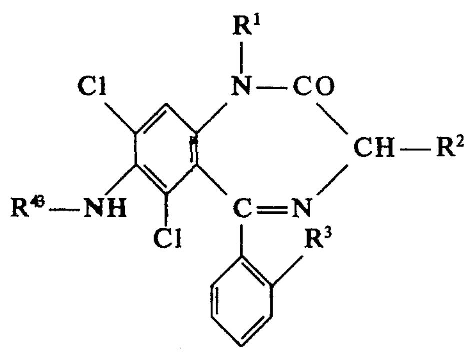

(Ib)

où $R^{43}$ représente un radical de formule

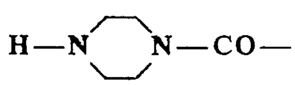

ou

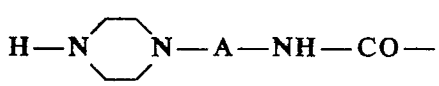

$R^1$, $R^2$, $R^3$ et A ont la signification donnée dans la revendication 1, ou

k) on cyclise un dérivé benzophénone de formule générale

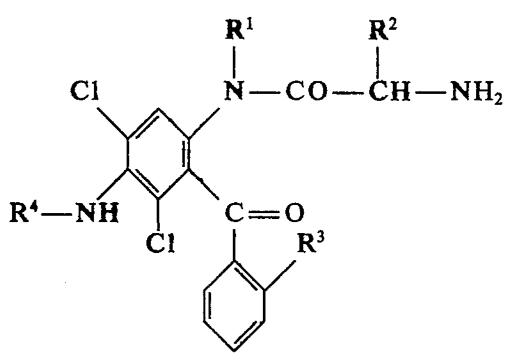

(XIV)

où $R^1$, $R^2$, $R^3$ et $R^4$ ont la signification donnée dans la revendication 1, ou

l) on transforme un dérivé de benzodiazépine de formule générale I en un sel d'addition d'acide

pharmaceutiquement acceptable.

18. Médicament contenant un composé selon l'une des revendications 1 à 9 ou un de ses sels pharmaceutiquement acceptables.

19. Médicament antagoniste de l'aldostérone contenant un composé selon l'une des revendications 1 à 9 ou un de ses sels d'additions d'acides pharmaceutiquement acceptables.